# EUROPÄISCHE PATENTANMELDUNG

(11) **EP 1 241 267 A2**
(43) Veröffentlichungstag der Anmeldung: **18.09.2002**
(21) Anmeldenummer: 02005362.5
(22) Anmeldetag: 14.03.2002
(51) Int. Cl.: C12Q 1/68

(54) **Enzym-Aktivitätstest mittels fluoreszenzmarkierter Oligonukleotid-Substrate**

(30) Priorität: 14.03.2001 EP 01106265
(71) Anmelder: Viscum AG, 64673 Zwingenberg (DE)
(72) Erfinder: Aygün, Hüseyin, 60322 Frankfurt (DE); Wojczewski, Sylvia, 65812 Bad Soden (DE)
(74) Vertreter: VOSSIUS & PARTNER

(57) **Zusammenfassung**

Die Erfindung betrifft ein Verfahren zum Nachweis einer Substanz mit einer enzymatischen Aktivität, die zu einer sequenzspezifischen Depurinierung oder Depyrimidinierung einer Nukleinsäure ohne Strangabbruch führt, oder mit einer sequenzspezifischen Endonucleaseaktivität, in einer Probe, wobei (a) die Probe (aa) mit einem Substrat in Kontakt gebracht wird, das (i) drei Nukleinsäuresequenzabschnitte (A, B und C) aufweist, wobei der Nukleinsäuresequenzabschnitt B zwischen den Abschnitten A und C liegt die Nukleinsäuresequenzabschnitte A und C Sequenzen aufweisen, die komplementär zueinander sind und somit unter physiologischen Bedingungen miteinander hybridisieren und dadurch das Substrat eine Haarnadelstruktur ausbildet und wobei der Nukleinsäuresequenzabschnitt B oder die Nukleinsäureabschnitte A und C nach Hybridisierung ein Erkennungsmotif für die Aktivität enthalten; und (ii) ein Fluorophor-Quencher-Paar aufweist, wobei das Fluorophor kovalent mit Sequenzabschnitt A oder C verbunden ist und der Quencher kovalent mit dem anderen Sequenzabschnitt (C oder A) verknüpft ist und wobei Fluorophor und Quencher sich bei der Hybridisierung der DNA-Sequenzen in räumlicher Nähe zueinander befinden, so daß die vom Fluorophor emittierte Lichtenergie vom Quencher gequencht wird; und (ab) sofern die Substanz eine Substanz mit einer enzymatischen Aktivität ist, die zu einer sequenzspezifischen Depurinierung oder Depyrimidinierung einer Nukleinsäure ohne Strangbruch führt, mit einem Agens in Kontakt gebracht wird, das das Substrat spezifisch an einer Nukleotidposition im Bereich des Nukleinsäuresequenzabschnitts B spaltet, die durch die Substanz depuriniert oder depyrimidiniert wird; und (b) anhand der Emission der Fluorphorspezifischen Lichtenergie ermittelt wird, ob eine Substanz mit der genannten Aktivität in der Probe vorhanden ist. Vorzugsweise enthält die zu testende Probe ein Ribosomen-inaktivierendes Protein (RIP), in einer besonderen Ausführungsform Mistellektin. Ferner betrifft die Erfindung einen Test-Kit zur Ausführung des Verfahrens welcher das spezifisch mit Fluorophor und Quencher markierte Substrat, die chemischen Spaltungsreagenzien sowie gegebenenfalls die für die Reaktionen vorbereiteten Reaktionsgefäße enthält.

## Beschreibung

Die Erfindung betrifft ein Verfahren zum Nachweis einer Substanz mit einer enzymatischen Aktivität, die zu einer sequenzspezifischen Depurinierung oder Depyrimidinierung einer Nukleinsäure ohne Strangabbruch führt, oder mit einer sequenzspezifischen Endonucleaseaktivität, in einer Probe, wobei (a) die Probe (aa) mit einem Substrat in Kontakt gebracht wird, das (i) drei Nukleinsäuresequenzabschnitte (A, B und C) aufweist, wobei der Nukleinsäuresequenzabschnitt B zwischen den Abschnitten A und C liegt, die Nukleinsäuresequenzabschnitte A und C Sequenzen aufweisen, die komplementär zueinander sind und somit unter physiologischen Bedingungen miteinander hybridisieren und dadurch das Substrat eine Haarnadelstruktur ausbildet und wobei der Nukleinsäuresequenzabschnitt B oder die Nukleinsäureabschnitte A und C nach Hybridisierung ein Erkennungsmotif für die Aktivität enthalten; und (ii) ein Fluorophor-Quencher-Paar aufweist, wobei das Fluorophor kovalent mit Sequenzabschnitt A oder C verbunden ist und der Quencher kovalent mit dem anderen Sequenzabschnitt (C oder A) verknüpft ist und wobei Fluorophor und Quencher sich bei der Hybridisierung der DNA-Sequenzen in räumlicher Nähe zueinander befinden, so daß die vom Fluorophor emittierte Lichtenergie vom Quencher gequencht wird; und (ab) sofern die Substanz eine Substanz mit einer enzymatischen Aktivität ist, die zu einer sequenzspezifischen Depurinierung oder Depyrimidinierung einer Nukleinsäure ohne Strangbruch führt, mit einem Agens in Kontakt gebracht wird, das das Substrat spezifisch an einer Nukleotidposition im Bereich des Nukleinsäuresequenzabschnitts B spaltet, die durch die Substanz depuriniert oder depyrimidiniert wird; und (b) anhand der Emission der Fluorophorspezifischen Lichtenergie ermittelt wird, ob eine Substanz mit der genannten Aktivität in der Probe vorhanden ist. Vorzugsweise enthält die zu testende Probe ein Ribosomen-inaktivierendes Protein (RIP), in einer besonders bevorzugten Ausführungsform Mistellektin. Ferner betrifft die Erfindung einen Test-Kit zur Ausführung des Verfahrens, welcher das spezifisch mit Fluorophor und Quencher markierte Substrat, die chemischen Spaltungsreagenzien sowie gegebenenfalls die für die Reaktionen vorbereiteten Reaktionsgefäße enthält.

In ihrer natürlichen Umgebung, d.h. im lebenden Organismus vorkommende Enzyme weisen, sofern sie nicht reprimiert oder proteolytisch abgebaut werden, ein bestimmtes Aktivitätsmuster auf, das unter anderem durch die katalytische Aktivität gekennzeichnet wird. Werden Enzyme, beispielsweise für industrielle Zwecke aus ihrer natürlichen Umgebung heraus isoliert und gelagert, so kann das mit einem Verlust an katalytischer Aktivität einhergehen. So ist beispielsweise für kommerziell verfügbare Restriktionsendonukleasen vom Typ II bekannt, daß die meisten kühl gelagert werden müssen. Ein Aufbewahren über einen längeren Zeitraum bei Raumtemperatur geht mit einem dramatischen Aktivitätsverlust einher.

Eine Bestimmung der Aktivität von enzymatischen Aktivitäten ist selbstverständlich nicht nur für Restriktionsenzyme erwünscht. Im Prinzip sind bei der Ermittlung enzymatischer Aktivitäten zwei Arten von Fragen von Interesse. Die eine hinterfragt, inwieweit eine enzymatische Aktivität, die bekanntermaßen in einer Probe vorhanden war, in dieser noch vorhanden ist (quantitativer Ansatz). Davon zu unterscheiden ist die Problemstellung, daß in einer Probe unbekannter oder teilweise unbekannter Konsistenz gemessen werden soll, ob hier bestimmte enzymatische Aktivitäten überhaupt vorhanden sind (qualitativer Ansatz). In diesem Zusammenhang ist vorstellbar, daß beispielsweise Bodenproben auf das Vorhandensein einer oder mehrerer interessierender biologischer Aktivitäten untersucht werden sollen. In vielen Fällen wird eine Kombination aus quantitativem und qualitativem Ansatz gewünscht sein. Einige Enzyme von besonderem Interesse werden nachstehend diskutiert. Das besondere Interesse ist unter anderem darauf begründet, daß manche dieser Enzyme, gegebenenfalls in modifizierter Form, klinisch relevant sein könnten.

Eine Aktivitätscharakterisierung von Molekülen mit N-Glycosidase Aktivität (AMP Nucleosidase (Mentch et al., 1987), AMP Deaminase (Meyer et al., 1989) die unspezifischen Nucleosid Hydrolasen (Goupaul et al., 1996), Purin Nucleosid Phosphorylase (Kline & Schramm, 1993) tRNA Transglcosylasen (Romier et al., 1996) und nicht zuletzt den Typ 1 und Typ 2 Ribosomen-inaktivierenden Proteinen (Übersichtsartikel: Barbieri et al., 1993) ist aufgrund des breiten Wirkspektrums und der Beteiligung vieler der o.g. Proteine an diversen wichtigen Stoffwechselprozessen von breitem Interesse. So wird eine Beteiligung der Purin Nucleosid Phosphorylase im Purin Recyceling beschrieben. Ein Gendefekt führt zu einer T-Zell-lmmundefizienz (Hershfield & Mitchell, 1995).

Typ 1 und Typ 2 Ribosomen Inaktivierende Proteine (RIP) werden schon seit langer Zeit wegen der beschriebenen Cytotoxizität (hervorgerufen durch die N-Glycosidase Aktivität) entweder direkt oder gekoppelt an monoklonale Antikörper oder Cytokine bei verschiedenen Indikationen eingesetzt (Barbieri et al., 1993).

Durch die enzymatische N-Glycosidase Aktivität wird aus der 28S rRNA spezifisch ein exponiertes Adenin aus dem GAGA Motiv aus einem konservierten Loop herausgeschnitten (A4324 bezogen auf rRNA aus Ratte; Endo et al., 1991). Es existieren trotz intensiver Untersuchungen und Kristallisationsstudien über den Reaktionsmechanismus verschiedene Vorschläge. Allen Vorschlägen gemein ist eine Protonierung der Austrittsgruppe (Adenin), im folgenden die Entstehung eines Oxocarbenium lons, welches durch einen negativ geladenen Aminosäurerest im aktiven Zentrum stabilisiert und im weiteren Verlauf durch Anlagerung von Wasser neutralisiert wird. Monzingo und Robertus (1992) beschreiben für Ricin eine Protonierung an Position N3 der Base durch den Aminosäurerest Arginin, während Huang et al. (1995) davon ausgehen, daß eine Protonierung an N7 unter Beteiligung der Aminosäure Glutamat erfolgt.

Eine Depurinierung führt zu einer Konformationsänderung in der Struktur der Ribosomen, welche bedingt, daß der für die Translationsreaktion benötigte Elongationsfaktor (eEF 2) nicht mehr binden kann. Der k_{cat} Wert des Ricins für Säuger-Ribosomen wird mit 1800 pro Minute beschrieben. Ein einziges Molekül wäre demnach in der Lage, eine Säugerzelle in kurzer Zeit abzutöten (Endo & Tsurugi, 1987a).

Zum therapeutischen Einsatz eines solch potenten Proteins ist ein verläßlicher Aktivitätstest vonnöten. Nur so kann eine definierte und immer wieder gleiche Dosis z.B. eines Wirkstoffes bereitgehalten werden. Es gibt im Stand der Technik grundsätzlich drei verschiedene Methoden, die rRNA-N-Glycosidase Aktivität zu beschreiben.

### (1) Quantifizierung der Inaktivierung von Ribosomen in zellfreien Proteinsynthese Systemen.

Im Prinzip werden bei diesen zellfreien Tests entweder Polysomen oder mRNA Moleküle zu isolierten Ribosomen gegeben und über den Einbau von radioaktiven Aminosäuren (z.B.: ³⁵Met oder ³⁵Cys) die Translationsrate bestimmt. In Abhängigkeit der Menge des während der Translation zugegebenen RIP kommt es nun zu einer Abnahme der Translationsaktivität. Zur Quantifizierung müssen hier häufig die gebildeten, radioaktiv markierten Proteine präzipitiert werden, was aufgrund der Varianz bei der Fällung zu Problemen bei der Auswertung führen kann (Pelham & Jackson, 1976; Gasperi-Campani et al., 1980; Franz et al., 1982; Barbieri et al., 1989).

Eine Verbesserung der o.g. Systeme stellte der gekoppelte Transkriptions/Translations Test dar, in dessen Verlauf an Kaninchen Reticulocyten Lysat nach Transkription des auf einem Plasmid codierten Luciferase Gens zu einer definierten Messenger RNA eine Translation dieser an den Ribosomen der Reticulocyten stattfindet. Eine Quantifizierung erfolgt im Anschluß durch Vermessung der Luciferase-Aktivität direkt im Überstand. Bei der CoA und ATP abhängigen Luciferin Umsetzung werden Photonen freigesetzt, die im Luminometer quantifiziert werden. Die Anzahl der freigesetzten Photonen korreliert dabei mit der Menge an translatierter Luciferase. Wie auch bei den oben beschriebenen Systemen wird in Abhängigkeit der zugesetzten RIPs die Translationsaktivität der Ribosomen und damit die Quantität an gebildeter Luciferase reduziert. Eine Auswertung der Daten erfolgt über einen Vergleich der aufgenommenen Meßwerte unter RIP-Einfluß mit einer nicht inhibierten Kontrolle (Langer et al., 1996; Skinner & Jackson, 1997). Die Vorteile sind zum einen das Arbeiten mit einer nichtradioaktiven Probe sowie das Arbeiten in einem was die mRNA anbelangt definierten System. Eine Limitation des Verfahrens ist jedoch durch die Qualität des Reticulocyten Lysates, welches in dem Test als eigentliches Substrat eingesetzt wird sowie die begrenzte Haltbarkeit des Lysates begründet.

### (2) Semiquantitative Analyse von rRNA-Spaltprodukten in Polyacrylamid-Gelen.

28S rRNA wird unter Einwirkung von RIP depuriniert. Die erfolgte Depurinierung der rRNA kann mittels anschließender Spaltung durch z.B. Anilin (im sauren pH-Bereich) oder durch milde alkalische Bedingungen und anschließende Auftrennung der Spaltprodukte im Polyacrylamid-Gel nachgewiesen und durch Mitführen eines geeigneten Standards über densitometrische Verfahren semiquantitativ erfaßt werden (Endo & Tsurugi, 1987b).

### (3) Direktes Vermessen von freigesetztem Adenin aus der 28S-rRNA

Zuerst seien hier die Methoden der HPLC-Chromatographie genannt. Eingesetzt werden insbesondere Substrate, von denen spezifisch ein Adenin aus dem GAGA Motiv durch spezifische Spaltung der N-glycosidischen Bindung herausgeschnitten wird. Hier erfolgt eine Quantifizierung des aus der 28S rRNA freigesetzten Adenins nach Umsetzung des Purins durch Chloracetaldehyd. Durch die chemische Modifikation entsteht das Fluorophor Ethenoadenin. Eine Quantifizierung des Ethenoadenins mittels Fluoreszenzdetektor und einer mitgeführten Ethenoadenin-Standardreihe erfolgte durch Peak-Integration und Auftragung der Peakfläche gegen die eingesetzte Menge an Ethenoadenin. Die Nachweisgrenze wird mit 2 ng modifiziertes Adenin angegeben (Zamboni et al. 1989). Ein Problem dieser Methode stellt die Modifikation des Adenins dar, welche nicht immer quantitativ ist und dadurch die Gefahr einer falschen Quantifizierung beinhaltet.

Eine weitere HPLC-Quantifizierungsmethode stellt die direkte Quantifizierung des freigesetzten Adenins dar (Chen et al. 1998). Die Autoren verwendeten anstelle der 28S rRNA synthetische RNA-Oligonukleotide einer variablen Länge (10 bis 20mer RNA-Nukleotide) als Substrat. Die Methode ist geeignet, Michaelis-Menten Kinetiken aufzunehmen. Eine Nachweisgrenze für das aus den verschiedenen Substraten freigesetzte Adenin mittels HPLC-Detektion bei 260 nm liegt bei etwa 1 µM.

Nachteile beider hier aufgeführten HPLC-Methoden sind die Nachweisgrenze von 1 µM Adenin (135 ng/ml) oder 2 ng Ethenoadenin, was damit verbunden ist, daß nur konzentrierte Enzymlösungen >10µg/ml vermessen werden können. Weiterhin von Nachteil sind die lange Laufzeit, sowie das Problem, daß man nur eine Reaktion (Probe) auf einmal messen kann (fehlende HTS Tauglichkeit).

Eine weitere Möglichkeit, das durch die N-Glycosidase-Aktivität von RIP freigesetzte Adenin direkt nachzuweisen und zu quantifizieren, beruht auf der von Brigotti et al. (1998) beschriebene Methode, die eine Weiterentwicklung der Beobachtungen von Barbieri et al. (1997) darstellt. Von Barbieri et al. (1996; 1997) konnte gezeigt werden, daß aus diversen DNA-Substraten (wie z.B. Hering Sperma DNA oder Poly-A-Substrate) dank der Polynukleotid:Adenosin Glycosidase Aktivität von RIP unspezifisch Adenine freigesetzt wurden. Zur Quantifizierung der RIP-Aktivität wurde nun von Brigotti et al. (1998) der Ansatz gewählt, ein 2250 bp großes Plasmid-Fragment von pBR322 mittels PCR unter Anwesenheit radioaktiv markierter [8-³H]dATP Nukleotide zu markieren und zu amplifizieren. Das so erhaltene Substrat ist reproduzierbar an den Adenin enthaltenen Bausteinen markiert und leicht darstellbar und wurde im folgenden als ein stabileres Substrat (als Ribosomen oder isolierte mRNA) für RIPs eingesetzt. Diese Methode ist grundsätzlich HTS tauglich, jedoch stellt die Darstellung des Substrates laut Aussagen der Autoren noch einen gewissen Engpaß dar.

Als ein weiteres Problem bei den von Brigotti et al. (1998) durchgeführten Versuchen stellte sich heraus, daß bei konstanter Substratkonzentration (PCR-Produkt) und steigender RIP Konzentration (0.01 pmol - 10 pmol) kein linearer Anstieg der Umsetzung (erhöhte Freisetzung der Radioaktivität) ergeben hat. Die Depurinierung des Substrates durch das N-glycosidisch aktive RIP erfolgt offensichtlich an mehreren Stellen entweder gleichzeitig oder nacheinander, was bedingt, daß die zugrundeliegende Kinetik kompliziert und sehr umfangreich ist. Eine Quantifizierung von RIP wird dadurch erschwert und muß empirisch von einer mitgeführten experimentellen Standardkurve ermittelt werden. Weiterhin geben die Autoren zu bedenken, daß beim Einsatz von 20 ng Substrat DNA ein unter den Reaktionsbedingungen vermutlich durch Präzipitation oder Oberflächenadsorption auftretender Verlust an Radioaktivität festgestellt wurde. Es wurde im folgenden versucht, diesen Verlust durch Einsatz von Faktor 15 mehr Substrat (300 ng) zu kompensieren, doch erscheint die finale lösliche Substratkonzentration variabel zu sein. Eine Validierung der Messung hinsichtlich der Wiederholpräzision wurde nicht gezeigt, ist jedoch für eine Verwendung des Tests für die Analyse von Wirkstoffen unabdingbar.

Interessanterweise werden von RIP nur wenige Ribosomen als Substrate erkannt. Endo & Tsurugi (1988) testeten verschiedene isolierte rRNAs. So wurden die untersuchten 28S rRNA aus Ratte, die 25S rRNA von Hefe, die 23S sowie die 16S rRNA aus *E*. *coli* an jeweils spezifischen Stellen (GAGA-Loops) erkannt und depuriniert, während andere im gleichen Substrat befindliche GAGA-Loops nicht depuriniert wurden. Die Autoren fanden, daß sich zwar der Kₘ-Wert der isolierten 28S rRNA nicht von dem Substrat im intakten 80S Ribosom unterscheidet, eine Bindung also mit der gleichen Affinität erfolgt. Im Gegensatz dazu ist der K_{cat}-Wert des isolierten Substrats mit 0.02 Ribosomen pro Minute im Vergleich zu 1777 Ribosomen pro Minute bei intakten Ribosomen dramatisch niedriger, was eventuell an einer unterstützenden Wirkung der Ribosomen assoziierten Proteine liegen könnte. Prokaryontische rRNA wird in intakten 70S Ribosomen nicht als Substrat erkannt. Nur in Abwesenheit der ribosomalen Proteine kann hier spezifisch ein Adenin abgespalten werden. Krawetz und Boston (2000) testeten ebenfalls verschiedene eukaryontische Ribosomen von Nager (Kaninchen), Weizenpathogen *Aspergillus flavus* und Mais-Pflanze auf die Umsetzbarkeit mit einem Typ 1 RIP und fanden heraus, daß die isolierte 28S rRNA des Freßfeindes Kaninchen am besten als Substrat erkannt wird (303 Moleküle pro Minute) während die 28S rRNA aus der Pflanze *Zea mays* (Faktor 4200 niedrigerer Umsatz) wie auch die 28S rRNA des Pilzes A. flavus (Faktor 240 niedrigerer Umsatz) nur sehr schlecht als Substrat erkannt wurden.

Ein synthetisches Substrat wird demnach vermutlich nie die hohen K_{cat}-Werte erreichen, wie sie für die intakten Ribosomen beschrieben wurden. Bisher wurden bereits synthetische Substrate zur Quantifizierung von N-Glycosidase Aktivitäten eingesetzt (Glück et al., 1992; Orita et al., 1996; Link et al., 1996; Chen et al., 1998). Da es sich bei diesen Oligonukleotid-Sequenzen um reine Ribonukleinsäure-Moleküle handelt, wurden Modifikation zur Stabilisierung der Struktur gegenüber RNasen eingeführt. Computergestützte Untersuchungen über die Bindung kleiner Hairpin-Strukturen an Ricin A (Olson, 1997) konnten zeigen, daß die primär in Frage kommenden, spezifischen Wechselwirkungen auf den Loop-Bereich eingeschränkt sind. Hier werden vor allem *van der Waals* sowie zahlreiche elektrostatische Wechselwirkungen zwischen beiden Interaktionspartnern gefunden. Außerhalb der Erkennungsequenz überwiegen im wesentlichen unspezifische Wechselwirkungsanteile mit dem Ribosephosphat Rückgrat. Spezifisch wurde von Glück et al. (1992) ein synthetisches RNA-Oligonukleotid eingesetzt, welches radioaktiv markierte Adenine eingebaut hatte. Dabei handelte es sich um ein 19-mer mit dem spezifischen GAGA-Motiv, abgeleitet aus einer Domäne der 16S rRNA aus *E*. *coli* (Moazed et al., 1986). Nach Depurinierung des Substrats wurde das abgespaltene Substrat mittels HPLC-Chromatographie aufgetrennt und die Adenin enthaltene Fraktion quantifiziert. Es wurde ein Kₘ-Wert von 5.7 µM mit einem K_{cat}-Wert von 0.01 pro Minute ermittelt. Mit einem 35-mer RNA-Oligonukleotid fanden sie ähnliche kinetische Werte (Kₘ-Wert von 13.6 µM mit einem K_{cat}-Wert von 0.02 pro Minute). Ein qualitativer Nachweis der Spaltung erfolgte durch Anilin-Spaltung an der depurinierten Stelle und einer anschließenden Auftrennung der beiden entstandenen Fragmente im Poiyacryiamid/Harnstoff Gel. Von Link et al. (1996) wurden ebenfalls einige RNA-Oligonukleotide als Substrate für Ricin eingesetzt. Ähnlich wie bei Glück et al. (1992) beschrieben, wurden auch bei diesen Oligonukleotiden niedrige katalytische Umsatzraten ("catalytic turnovers") im Bereich von 6.6 x10⁻⁵ bis 1x 10⁻⁴ Umsätze pro Ricin A-Molekül und Minute aufgenommen. Die niedrigen Umsatzraten werden von den Autoren mit einer Kompetition zwischen Depurinierung und Aufschmelzen der Oligonukleotid-Struktur diskutiert, wobei der Schwerpunkt ganz auf der Seite der Substrat-Schmelzens zu liegen scheint.

Weitere synthetische Substrate für Ricin wurden von Chen et al., (1998) beschrieben. Die Oligonukleotide, die von den Experimentatoren eingesetzt wurden, waren RNA-Oligonukleotide mit einem GAGA-Loop und unterschiedlich langen Stämmen. Die Kₘ-Werte der von Chen et al. eingesetzten Oligonukleotide lagen alle im einstelligen µM-Bereich, während die K_{cat}-Werte einen glockenförmigen Verlauf nehmen. Dabei wurde gezeigt, daß eine mittlere Länge des Stamms von der höchsten Umsatzgeschwindigkeit begleitet war (219/min). Als limitierend stellt sich bei der HPLC Analytik der massive Zeitaufwand bei der Probenvorbereitung wie auch der beschränkte Probendurchsatz dar. Eine Reaktion muß, um in detektierbare Bereiche des freigesetzten Adenins zu gelangen, etwa 1 bis 2 Stunden inkubieren. Anschließend muß die Probe noch der HPLC-Analytik unterzogen werden. Proben können nicht parallel vermessen werden sondern müssen zeitversetzt angesetzt werden, was die experimentelle Vorbereitungszeit extrem verlängert und kostenintensiv ist.

Noch bis heute werden die meisten Endonucleasen über die Menge Enzym quantifiziert, die die Spaltung von 1 µg DNA in 50 µl Volumen pro Stunde in den entsprechenden Puffern bewerkstelligt (entspr. 1 Unit). Eine Analyse der Umsetzung erfolgt dabei mittels Agarosegelelekrophorese. Es gibt wenige im Stand der Technik beschriebene Ansätze, dieses aufwendige und kostenintensive Verfahrendurch einfachere Protokolle zu ersetzen.

So wurde Endonuclease Aktivität des Restriktionsenzyms Pae R7 von Ghosh et al. mittels FRET-Technologie vermessen (1994). Hier wurden zwei komplementäre Oligonukleotide, die jeweils am 5'-Ende markiert waren, mit Fluorophor und Quencher gelabelt. Ein wesentlicher Nachteil dieser Methode ist, daß man sehr lange Oligonukleotide synthetisieren muß, damit sie nicht unter normalen Bedingungen z.B. 37 °C aufschmelzen. Auch dieses Verfahren ist somit mit erheblichem Material- und Kostenaufwand verbunden.

Die den Stand der Technik repräsentierenden Methoden beinhalten verschiedenartige Limitationen bezüglich der Kostenintensität, Reproduzierbarkeit, Tauglichkeit auf Hochdurchsatzanalyse von Proben, Validität und Substraterkennung.

Die der hier beschriebenen Erfindung zugrundeliegende Aufgabe war somit, diese Nachteile aus dem Stand der Technik zu überwinden und den von einem in bleibender Qualität bereitzuhaltenden Standard unabhängige, valide Nachweis einer enzymatischen Aktivität, die entweder eine Endonukleaseaktivität oder eine Depurinierungs-/Depyrimidierungsaktivitität aufweisen, zu gewährleisten. Diese Aufgabe wird durch die in den Ansprüchen gekennzeichneten Ausführungsformen gelöst.

Somit betrifft die Erfindung ein Verfahren zum Nachweis einer Substanz mit einer enzymatischen Aktivität, die zu einer sequenzspezifischen Depurinierung oder Depyrimidinierung einer Nukleinsäure ohne Strangabbruch führt, oder mit einer sequenzspezifischen Endonucleaseaktivität, in einer Probe, wobei (a) die Probe (aa) mit einem Substrat in Kontakt gebracht wird, das (i) drei Nukleinsäuresequenzabschnitte (A, B und C) aufweist, wobei der Nukleinsäuresequenzabschnitt B zwischen den Abschnitten A und C liegt, die Nukleinsäuresequenzabschnitte A und C Sequenzen aufweisen, die komplementär zueinander sind und somit unter physiologischen Bedingungen miteinander hybridisieren und dadurch das Substrat eine Haarnadelstruktur ausbildet und wobei der Nukleinsäuresequenzabschnitt B oder die Nukleinsäureabschnitte A und C nach Hybridisierung ein Erkennungsmotif für die Aktivität enthalten; und (ii) ein Fluorophor-Quencher-Paar aufweist, wobei das Fluorophor kovalent mit Sequenzabschnitt A oder C verbunden ist und der Quencher kovalent mit dem anderen Sequenzabschnitt (C oder A) verknüpft ist und wobei Fluorophor und Quencher sich bei der Hybridisierung der DNA-Sequenzen in räumlicher Nähe zueinander befinden, so daß die vom Fluorophor emittierte Lichtenergie vom Quencher gequencht wird; und (ab) sofern die Substanz eine Substanz mit einer enzymatischen Aktivität ist, die zu einer sequenzspezifischen Depurinierung oder Depyrimidinierung einer Nukleinsäure ohne Strangbruch führt, mit einem Agens in Kontakt gebracht wird, das das Substrat spezifisch an einer Nukleotidposition im Bereich des Nukleinsäuresequenzabschnitts B spaltet, die durch die Substanz depuriniert oder depyrimidiniert wird; und (b) anhand der Emission der Fluorophorspezifischen Lichtenergie ermittelt wird, ob eine Substanz mit der genannten Aktivität in der Probe vorhanden ist.

Im Zusammenhang mit der vorliegenden Erfindung bedeutet der Begriff "räumliche Nähe" den Abstand den Fluorophor und Quencher benötigen, um einen fluoreszenzfreien Enegietransfer (ohne Hintergrundsignale) vom Fluorophor auf den Quencher zu gewährleisten. Dieser Abstand ist für den Fachmann ohne weiteres ermittelbar. Er ist z.B. dann gegeben, wenn Fluorophor und Quencher mit bei einer Hybridisierung von DNA-Molekülen direkt miteinander hybridiserten Nukleotiden verknüpft sind.

Erfindungsgemäß wird somit ein Verfahren bereitgestellt, das für eine Vielzahl von enzymatischen Aktivitäten einen einfach durchzuführenden und genauen Nachweis erbringt. Wesentlich ist für das erfindungsgemäße Verfahren und damit für die verschiedenartigen zu testenden Enzyme, daß unter Testbedingungen ein Nukleinsäuredoppelstrang unter Einbeziehung der vorstehend als Sequenzabschnitte A und C bezeichneten Bereich gebildet wird, der einseitig in eine Schleifenstruktur einmündet, in der sich die Erkennungs- bzw. Spaltstelle (oder auch mehrere solcher Stellen) für die enzymatische Aktivität befindet. Die enzymatische Aktivität kann entweder zu einer sequenzspezifischen Depurinierung oder Depyrimidierung führen oder aber kann eine sequenzspezifische Endonukleaseaktivität sein. Sofern die Aktivität eine sequenzspezifische Depurinierung oder Depyrimidierung umfaßt, beinhaltet das erfindungsgemäße Verfahren einen Spaltungsschritt mit einem Agens an der Nukleotidposition, die durch die Aktivität depuriniert oder depyrimidiert wurde. Bei Nachweis der Endonukleaseaktivität entfällt dieser Schritt, da die Endonuklease das Substrat direkt spaltet.

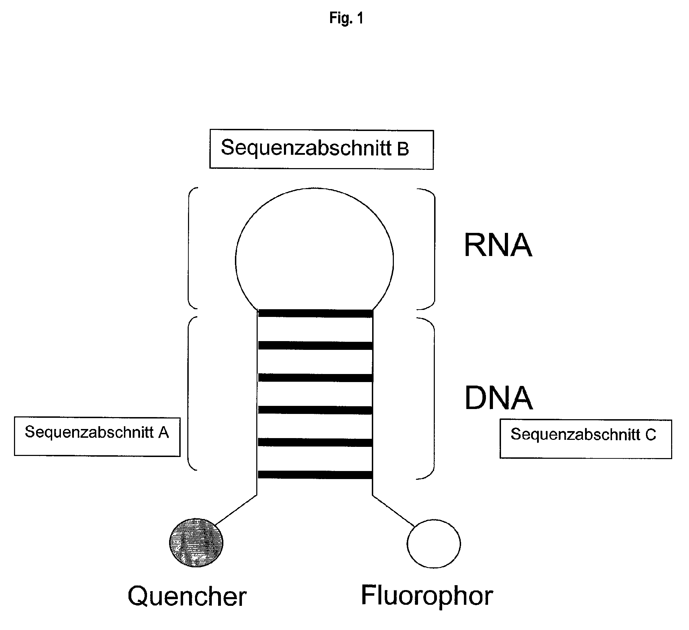

Wie erwähnt, geht das erfindungsgemäße Verfahren zur Detektion der genannten enzymatischen Aktivitäten von synthetisch aufgebauten Substraten aus, die unter physiologischen Bedingungen ein Oligonukleotid-Hairpin-System (OHS) ausbilden. Dabei tragen die als Sequenzabschnitte A und C bezeichneten Bereiche, z.B. an beiden Enden, Markierungen. Als Markierungen werden hierbei ein Flurophor und ein Quencher eingesetzt (schematisch in Fig. 1 dargestellt). Der Quencher ist dabei so ausgewählt, daß er die Anregungsenergie des Fluorophors größtenteils aufnimmt und dadurch die Eigenfluoreszenz abschwächt (FRET; Fluoreszenz-Energie-Transfer). Das erfindungsgemäße Verfahren wird nachstehend anhand einer bevorzugten Ausführungsform (wobei das zu testende Enzym das Substrat depuriniert) näher beschrieben: Wird ein solches OHS mit DNA als Sequenzabschnitte A und C als Substrat für N-Gycosidasen eingesetzt, wird die charakteristische Ribonukleinsäure-Erkennungssequenz 5'-GAGA-3' (enthalten im Sequenzabschnitt B) vom Enzym erkannt und spezifisch deadenyliert (5'-G_GA-3'). Der zurückbleibende depurinierte-Bereich läßt sich in einem zweiten Schritt selektiv mit Hilfe eines Agens, vorzugsweise von N-(4-Aminobenzoyl)-glycin oder 4-Aminohippursäure spalten. Nach der Spaltungsreaktion des OHS kommt es zu einer Trennung des 5' Bereichs vom 3' Bereich. Die Fluoreszenzabsorption durch den Quencher wird unterbrochen und die Gesamtfluoreszenz der Reaktion nimmt deutlich und reproduzierbar zu. Da diese Fluoreszenzzunahme proportional zu der enzymatischen Umsetzung des OHS ist, können über eine mitgeführte Eichreihe Rückschlüsse auf die Enzymkonzentration bzw. Aktivität von z.B. N-Glycosidasen durchgeführt werden, sofern eine quantitative Auswertung gewünscht ist. Wird in einer anderen bevorzugten Ausführungsform auf Anwesenheit einer sequenzspezifischen DNA Endonuklease getestet, so kann der oben genannte Versuchsaufbau mit folgenden Abwandlungen gewählt werden. Das OHS besteht im Sequenzabschnitt B entweder aus RNA oder wie in den Sequenzabschnitten A und C aus DNA. Die Sequenzabschitte A und C enthalten nach Hybridisierung ein Erkennungsmotiv für Restriktionsendonukleasen (palindromische Sequenz), die von einem zu testenden Restriktionsenzym gespalten wird. Sofern auf die Anwesenheit bzw. Aktivität von mindestens zwei solcher Restriktionsenzyme getestet werden soll, können die Sequenzabschitte A und C nach Hybridisierung auch mehrere unterschiedliche Palindrome enthalten. Beispiele hierfür sind die Sequenzen CCGG (Hpa II), GGCC (Hae III), GGATCC (Bam HI), GAATTC (Eco RI) und AAGCTT (Hind III). Der zweite Schritt der Spaltung mit einem spezifischen Agens wie 4-Aminohippursäure entfällt. Die Vorteile gegenüber dem von Gosh et al. beschriebenen Verfahren liegen insbesondere in der erwarteten verminderten Abhängigkeit von thermodynamischen Einflüssen und damit einer verbesserten Anwendbarkeit unter Laborbedingungen.

Ferner ist mit dem obigen Verfahrensaufbau (Abschnitte A und C: DNA, Abschnitt B: RNA) die Aktivität spezifisch RNA spaltender Enzyme wie Ribozyme möglich. Wird in Abschnitt B DNA eingesetzt, so können auch einzelsträngige DNA spaltende Endonukleasen getestet werden.

Im Prinzip läßt sich, wie vorstehend erläutert, mit dem erfindungsgemäßen Verfahren testen, ob in einer Probe mit nicht oder nicht vollständig bekanntem Inhalt überhaupt eine der obengenannten enzymatischen Aktivitäten vorhanden ist. Darüber hinaus ist es prinzipiell möglich zu untersuchen, inwieweit in einer Probe, die ein bestimmtes Enzym enthält, dieses Enzym noch aktiv ist. So können beispielsweise Batches kommerziell erhältlicher Restriktionsenzyme einfach, schnell und genau auf verbleibende Aktivität getestet werden. Um ein solches Verfahren in einem Labor im hohen Durchsatz durchführen zu können, werden neben den typischen Pipettierhilfen und Inkubatoren (Wasserbäder) auch ein Fluoreszenzdetektionssystem (z.B. Fluostar von BMG Laboratories) benötigt, das neben einem Termperiersystem auch mindestens eine automatische Pumpe zum Start der Reaktionen beinhalten sollte.

Ein wesentlicher Aspekt bei der Ausgestaltung des Substrats ist die räumliche Nähe von Fluorophor und Quencher unter physiologischen Bedingungen, wenn also die OHS-Konfiguration eingenommen wird. Solche Bedingungen werden beispielsweise in 0,85% NaCI, phosphatgepuffert bei pH 5-9 und 37°C erreicht. Sie werden auch erreicht in kommerziell erhältlichen Enzympuffern, z.B. basierend auf TRIS, Bis-TRIS, Phosphat, MES, MOPS etc., die gegebenenfalls vor dem Einsatz noch verdünnt werden müssen. Weitere Konditionen, die im erfindungsgemäßen Verfahren eingesetzt werden können, sind in den beigeschlossenen Beispielen dargestellt. Sie können vom Fachmann ferner anhand seines Fachwissens und der bekannten Literatur für das zu testende Enzym eingesetzt werden; vgl. z.B. Sambrook et al. (1989). Fluorophor und Quencher können innerhalb des hybridisierenden Bereichs der Sequenzabschnitte A und C - z.B. mittig - an entsprechend lokalisierte Nukleotide gekoppelt sein. Sie können jedoch - bevorzugt - auch am 3' und 5' Ende gekoppelt sein.

Die Bereiche A und C können vollständig oder teilweise miteinander hybridisieren. Wichtig ist jedoch, daß sie bei den oben angegebenen Bedingungen ein Hybrid ausbilden. Bevorzugt im Sinne des erfindungsgemäßen Verfahrens ist, daß die Bereiche A und C vollständig miteinander hybridisieren. Von der vorliegenden Erfindung umfaßt ist jedoch auch, daß die Bereiche A und C Basenfehlpaarungen aufweisen, solange die Hybridisierung unter den obengenannten Bedingungen erfolgt. Sofern Bereiche eingesetzt werden sollen, die bei Hybridisierung zu Basenfehlpaarungen führen, ist der Fachmann in der Lage, die Puffer-, zeitlichen und Temperaturbedingungen so zu justieren, daß für die Zwecke des erfindungsgemäßen Verfahrens eine OHS-Ausbildung zustande kommt. Die entsprechenden Bedingungen lassen sich für den Fachmann beispielsweise aus Standardwerken wie Hames & Higgins, "Nucleic acid hybridisation, a practical approach", IRL Press, Oxford, Washington D.C. 1985, vgl. insbesondere Kapitel 1, "Hybridisation Strategy" von R.J. Britten und E.J. Davidson ohne weiteres ableiten. In einer anderen Ausführungsform weisen die Bereiche A und C an ihrem dem Bereich B abgewandten Ende Sequenzbereiche auf, die nicht oder unter physiologischen Bedingungen nicht miteinander hybridisieren. Sofern die genannten Bereiche A und C solche Sequenzbereiche aufweisen, muß allerdings gewährleistet sein, daß insgesamt eine Hybridisierung der Bereiche A und C unter den genannten Bedingungen noch zustande kommt. Eine entsprechende Zusammensetzung des Substrats ist vom Fachmann ebenfalls ohne weiteres ermittelbar. Sofern diese Ausführungsform gewählt wird, ist nicht bevorzugt, daß Fluorophor und Quencher endständig positioniert (d.h. am 3'- und 5'-Ende des Substrats) sind. Auch diese Ausführungsformen bilden unter physiologischen Bedingungen Strukturen aus, die im Sinne der Erfindung als Haarnadelstrukturen bezeichnet werden. Bevorzugt ist ferner, daß das Substrat-Nukleinsäuremolekül A und/oder C ggf. in Kombination mit dem anhängigen Teilbereich B nach der Spaltung eine eigenständige meßbare Funktion ausführt, die zusätzliche Informationen zu der von der Fluoreszenz generierten Information liefert. Diese zusätzliche Funktion kann beispielsweise eine von der Fluoreszenzmessung unabhängige indirekte Messung auf Vorhandensein der hinterfragten enzymatischen Aktivität liefern. So umfaßt die vorliegende Erfindung Ausführungsformen, bei der der Sequenzbereich A oder C ggf. in Kombination mit dem anhängigen Teilbereich B nach der Spaltung ein Aptamer bildet, das durch Bindung an einen geeigneten Bindungspartner nachgewiesen werden kann. Sofern das Substrat vollständig oder in den Bereichen A und/oder C aus RNA besteht, können die Bereiche A und/oder C ggf. in Kombination mit dem anhängigen Teilbereich B nach Spaltung eine Ribozymstruktur aufweisen, deren Aktivität in einem gleichzeitig oder zeitversetzt durchgeführten Verfahrensschritt nachgewiesen werden kann.

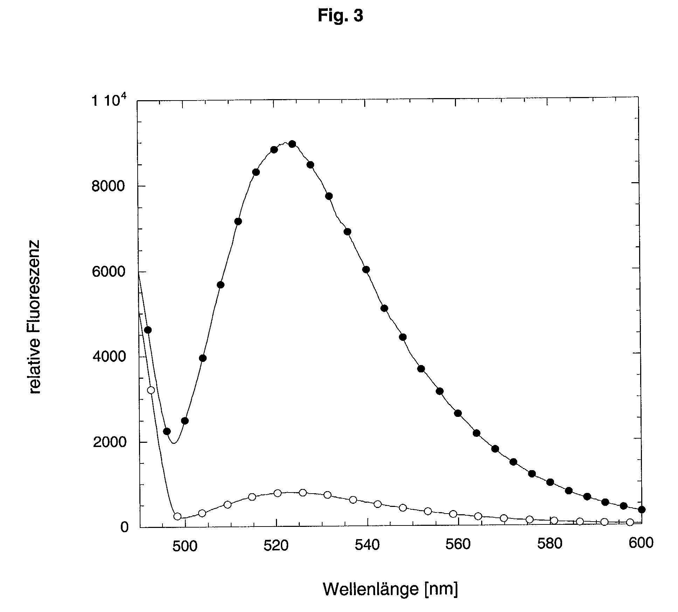

Ein wichtiger Punkt beim Einsatz von gequenchten Fluorophoren sind die maximal erreichbaren Fluoreszenzamplituden (Beispiel 4). Diese können durch Differenzmessung von gespaltenem (Fluorophor / Quencher-Paar ist räumlich getrennt und damit nicht mehr gequencht) zu ungespaltenem (gequencht = Hintergrundfluoreszenz) OHS erhalten werden (Fig. 3; gespalten ●; ungespalten ○). Das Differenzspektrum erbrachte bei allen erfindungsgemäß getesteten Substraten ein einheitliches Emissionsmaximum (ΔEₘₐₓ) um 523 nm. Die gefundenen Amplitudendifferenzen (F_{gespalten}-F_{ungespalten}) lagen je nach untersuchtem OHS bei Faktoren zwischen 3 und 10 (bei 523 nm), was für den Einsatz der OHS als Substrate zur Quantifizierung der genannten Enzymaktivitäten und insbesondere der N-Glycosidase Aktivität in jedem Falle als ausreichend zu werten ist.

Temperatur und Dauer der einzelnen Verfahrensschritte können vom Fachmann je nach gewünschtem Test eingestellt werden und/oder optimiert werden. Üblicherweise werden die Restriktionsanalysen bei einer Temperatur von 37 °C vermessen, da dies den physiologischen Bedingungen entspricht. Ein Vermessen von Enzymreaktionen kann aber auch bei tieferen Temperaturen (z.B. 25 °C) erfolgen. Entscheidend ist, daß man bei einem gewünschten Vergleich verschiedener Enzyme die Reaktionsbedingungen im wesentlichen identisch hält, da davon auszugehen ist, daß die Temperaturabhängigkeit der Enzymreaktionen einer Optimumskurve folgt.

Ein entscheidender Vorteil des erfindungsgemäßen Verfahrens gegenüber den bisherigen Systemen aus dem Stand der Technik besteht in seiner Robustheit und der Möglichkeit, die Messung (Fluoreszenzzunahme) sowohl während der Reaktion *(online)* als auch im Anschluß an die enzymatische Umsetzung *(postenzymatisch)* zu messen.

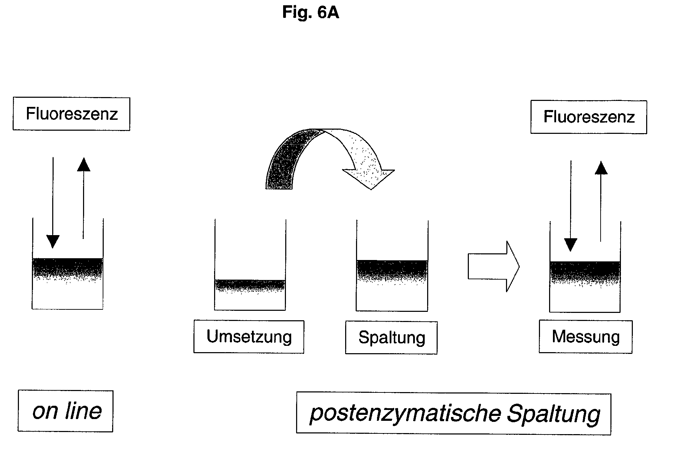

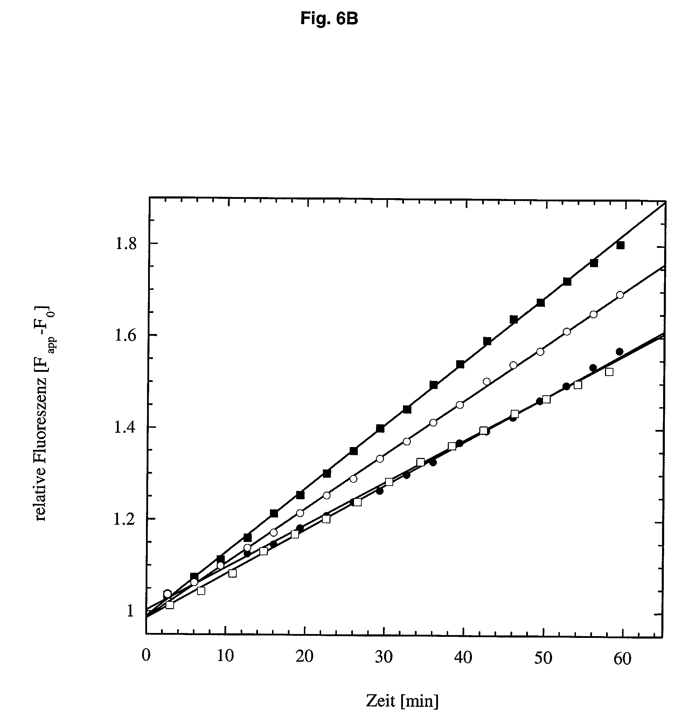

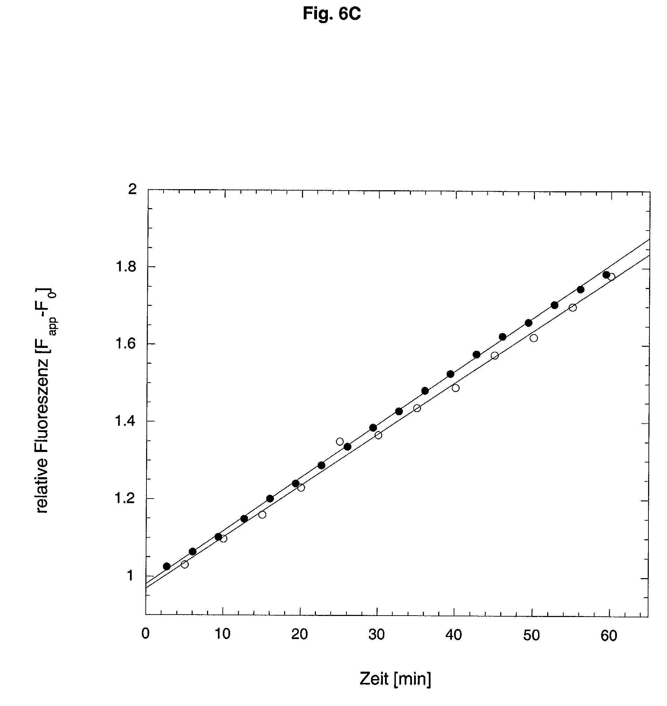

Die erfindungsgemäß doppelt markierten Substrate (beispielhaft dargestellt durch OHS-3 bis OHS-9) lassen sich in einem Assay auf Aktivitäten, die zu einer sequenzspezifischen Depurinierung oder Depyrimidierung ohne Strangbruch führen, ganz unterschiedlich einsetzen (Beispiel 7; Fig. 6A). Zum einen kann die Spaltungsreaktion direkt in Gegenwart von spaltendem Agens, z.B. 4-Aminohippursäure durchgeführt *(online)* werden. Eine andere Möglichkeit bietet sich, wenn die enzymatische Reaktion von der Fragmentierung getrennt wird *(postenzymatisch).* Beide Einsatzmöglichkeiten haben ihre Vorteile. Wichtig bei der Verwendung zur *online* Detektion ist das Verhältnis der beiden Geschwindigkeitskonstanten sowie die Kompatibilität von Enzym, vorzugsweise N-Glycosidase und spaltendem Agens, z.B. 4-Aminohippursäure. Dabei sollte die Spaltungsreaktion nicht geschwindigkeitslimitierend für die Gesamtreaktion sein, um die enzymatische Umsetzung hinreichend gut kinetisch aufzulösen. Beispielhaft durchgeführte Untersuchungen mit den nachfolgend ausführlicher diskutierten Ausführungsformen 4-Aminohippursäure als Spaltagens und rViscumin als Enzym belegen, wie das erfindungsgemäße Verfahren auf online bzw. postenzymatische Bedingungen optimal eingestellt werden kann: Untersuchungen bei unterschiedlichen 4-AHIP Konzentrationen konnten tatsächlich zeigen (Beispiel 7), daß Konzentrationen oberhalb sowie unterhalb von 12.5 mM 4-Aminohippursäure die Gesamtreaktion deutlich verlangsamen (Fig. 6B; geschlossene Kreise: 5 mM; geschlossenen Quadrate: 12.5 mM; offene Kreise: 25 mM und offene Quadrate: 50 mM). Wie aus weiteren Messungen hervorgeht, ist die Spaltungsreaktion zusätzlich geschwindigkeitslimitierend. Dieser Nachweis konnte anhand vergleichender Messungen mit bereits enzymatisch vorinkubierten Substraten erbracht werden (Fig. 6C; offene Kreise: *online* und geschlossene Kreise: *postenzymatisch).* Die Steigung der Geraden, welche die Umsatzgeschwindigkeit repräsentiert, ist in beiden betrachteten Fällen gleich. Ein weiterer Vorteil der *postenzymatischen* Prozeßführung liegt in den günstigen Meßbedingungen für das Reportermolekül Fluorescein (pH 3.0 bis pH 4.0). Gegenüber der *postenzymatischen* Detektion wird bei der *online* Spaltung gleichzeitig mit dem enzymatischen Inkubationsschritt mit 4-Aminohippursäure gespalten. Durch die Entkopplung von Enzymreaktion und Spaltungsreaktion treten bei dem *postenzymatischen* Ansatz keine störenden Einflüsse für die N-Glycosidase auf. Die umgesetzten Substrate lassen sich außerdem in Gegenwart wesentlich höherer 4-Aminohippursäure Konzentrationen (100 mM) durchführen.

Ausgehend von dem *postenzymatischen* Verfahren wurden anschließend kinetische Messungen unter optimierten Bedingungen mit unterschiedlich hohen Enzymkonzentrationen durchgeführt (Beispiel 8). Bei Vorversuchen konnte von allen beschriebenen Substraten mit OHS-8 die besten Ergebnisse erhalten werden. Interessanterweise unterscheidet sich OHS-8 von den Oligonukleotiden OHS-3 bis OHS-6 nur durch die obere Hälfte des Oligonukleotidstammes. Dieser Bereich ist bei OHS-8 deutlich AT-reicher und an das natürliche Substrat E16S (Endo & Tsurugi, 1988) angepaßt. Diese Ergebnisse stehen nicht im Einklang mit den von Chen et al. (1998) publizierten Daten mit der Ricin A-Kette. Hier konnten mit CG-reichen Stämmen Umsatzraten von bis zu 200 min⁻¹ gefunden werden. Es darf dabei aber nicht vergessen werden, daß es sich bei OHS-3 bis OHS-6 um chimäre Systeme aus DNA Stämmen und RNA-Loops handelt, die eine veränderte Konformation im Stamm aufweisen können.

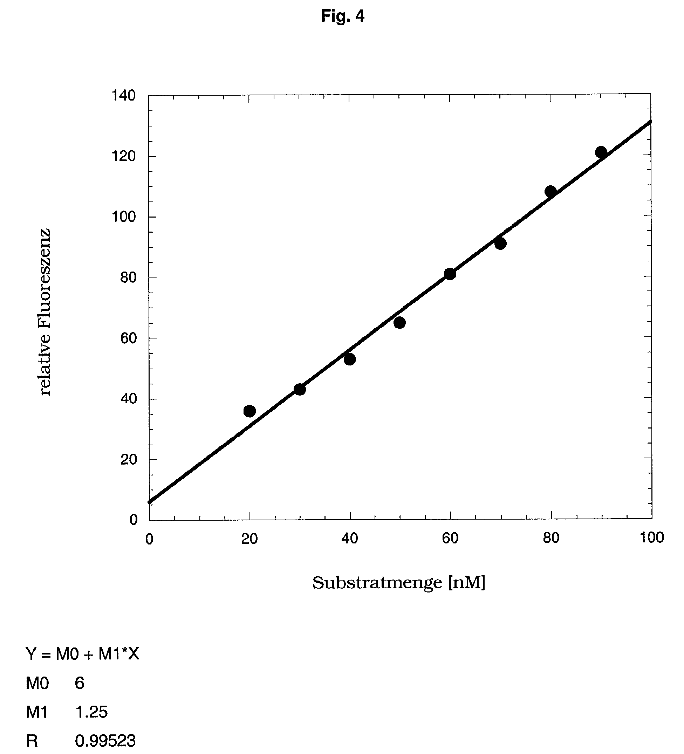

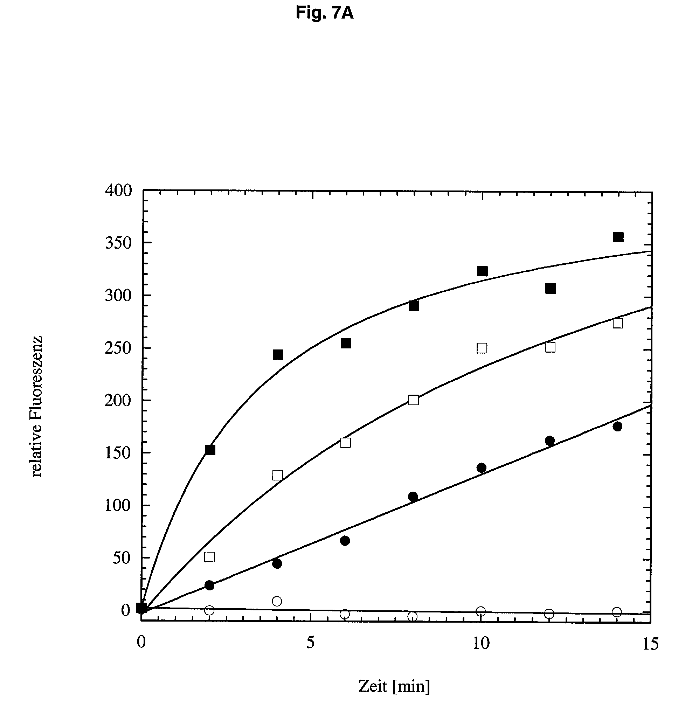

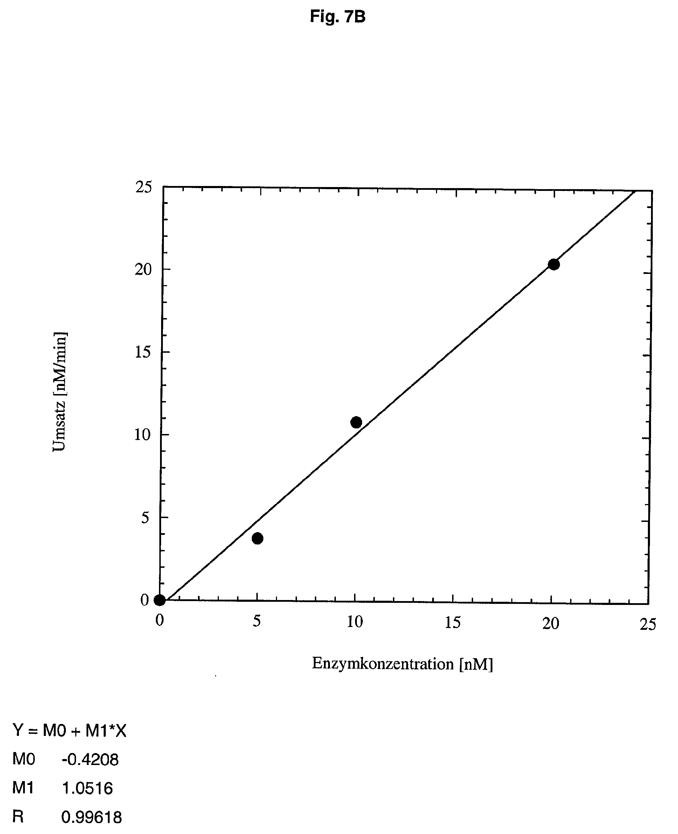

Bei den kinetischen Messungen wurde eine definierte Menge OHS-8 (333 nM) mit unterschiedlichen Enzymkonzentrationen inkubiert (Beispiel 8). Die erhaltenen Fluoreszenzmessungen zeigen bereits ganz eindeutig die Abhängigkeit des Fluoreszenzanstiegs von der eingesetzten Enzymkonzentration (Fig. 7A). Ohne Einsatz von Enzym (offene Kreise) erhält man über die Zeit keine Zunahme des Fluoreszenzsignals. Bei Einsatz von 5 nM rViscumin (geschlossene Kreise) erhält man über den betrachteten Zeitraum einen linearen Anstieg. Eine Verdoppelung der Enzymkonzentration auf 10 nM (offene Quadrate) führt wie erwartet zu einem steileren Anstieg der Fluorszenz über die Zeit. Allerdings deutet bereits etwa 5-10 min der Inkubation eine Sättigung der Fluoreszenzintensität an. Diese Beobachtung wird bei Einsatz von 20 nM rViscumin noch verstärkt (geschlossene Quadrate). Eine lineare Abhängigkeit der Fluoreszenz von der Zeit findet man hier nur noch in den ersten 5 min der Reaktion. Die Daten konnten über die mitgeführte Eichreihe (Fig. 4) in Konzentrationen umgerechnet werden. Die innerhalb der ersten 4 min erhaltenen Werte dienten nach linearer Regression für die Ermittlung der Umsatzraten (Steigung). Trägt man diese graphisch gegen die eingesetzte Enzymkonzentration auf, läßt sich ein lineares Verhalten beobachten (Fig. 7B). Bei Einsatz von 10 nM rViscumin errechnet sich so eine Umsatzrate von 10.05 nM OHS-8 Molekülen pro Minute. Diese Daten belegen, daß mit sehr geringen Abweichungen (R=0.996) unbekannte Enzymkonzentrationen bzw. Aktivitäten in niedrigen Konzentrationsbereichen quantifiziert werden können.

Das hier beschriebene Verfahren kann, im Gegensatz zu vielen im Stand der Technik beschriebenen Verfahren, eingesetzt werden, um Proben im Hochdurchsatz auf die vorgenannten enzymatischen Aktivitäten hin und vorzugsweise auf N-Glycosidase Aktivität zu untersuchen. Damit kann es gelingen, neue potentiell therapeutisch einsetzbare N-Glycosidasen zu finden und zu charakterisieren. Bisher beschriebene Verfahren ließen nur Einzelbestimmungen zu. Ein vermehrter Durchsatz von Proben wäre mit diesen Methoden nur mit einem erheblichen Zeitaufwand zu realisieren gewesen.

Die Vorteile zur Bestimmung der Aktivität von Endonukleasen liegen auf der Hand. Endonukleasen wie z.B. Restriktionsenzyme werden von verschiedenen Anbietern mit einer Aktivitätseinheit beschrieben, die in der Handhabung sehr aufwendig ist. Eine Stabilitätsverfolgung der Restriktionsenzyme ist demnach sehr kostenintensiv und wird in einer Qualitätskontrolle selten mit angeboten. Das Durchmustern von Mikroorganismen nach neuen Endonukleaseaktivitäten erweist sich mit den herkömmlichen Tests ebenfalls als schwierig. Ein modifizierter Test mit einem definierten Substrat bringt hier bei der Findung und Charakterisierung von Restriktionsenzymen entscheidende Vorteile. So können auch synthetische Substrate mit Sequenzen, die bisher nicht von Restriktionsenzymen erkannt werden, mit Hilfe dieses Testsystems identifiziert werden.

Von Wichtigkeit sind diese Vorteile des erfindungsgemäßen Verfahrens ferner und insbesondere für die Beschreibung der konsistenten Herstellung sowie die klinische Entwicklung der N-Glycosidase Mistellektin oder des rViscumin, da die beschriebenen Aktivitäten der Moleküle (z.B. Aktivierung von verschiedenen Zellpopulationen wie den "Large Granular Lymphocytes" (LGL) oder von natürlichen Killerzellen (NK-Zellen) (Hajto et al., 1989) sowie Induktion von Cytokinen wie TNFα und IFNγ (Möckel et al., 1997) oder Oberflächenmarkern wie CD-25 und HLA-DQ (Beuth et al., 1992) sehr stark dosisabhängig sind. Die Abhängigkeit von der Dosis kann durch eine Gaußsche Verteilung beschrieben werden. Für eine genaue Dosierung des Wirkstoffes ist es daher nötig, die Aktivität der toxischen Komponente genau zu quantifizieren.

In einer bevorzugten Ausführungsform des erfindungsgemäßen Verfahrens ist der Nachweis ein qualitativer Nachweis.

Der qualitative Nachweis ist der mit dem erfindungsgemäßen Verfahren am einfachsten zu führende Nachweis, da er auch mit dem in dieser Anmeldung beschriebenen nicht optimalen Versuchsbedingungen zuverlässig zum Erfolg führt.

In einer weiteren bevorzugten Ausführungsform des erfindungsgemäßen Verfahrens ist der Nachweis ein quantitativer Nachweis.
Bei einer Quantifizierung der Substratumsetzung erfolgt die Korrelation zwischen umgesetztem Substrat und Fluoreszenz (Findung des linearen Bereichs) über die Messung einer zuvor vollständig durch chemische Umsetzung gespaltenen Referenzprobe. Zu diesem Zweck wurden bei allen experimentell verwendeten Substraten alkalische Spaltungen vorgenommen (Beispiel 5) und entsprechend verdünnt (Aufnahme einer Eichreihe). Die erhaltenen Werte konnten über große Bereiche linear angeglichen werden (Fig. 4). Messungen, die an unterschiedlichen Tagen mit dem gleichen Substrat vorgenommen wurden, erbrachten reproduzierbare Eichgeraden. Diese Daten unterstreichen die Robustheit des erfindungsgemäßen Systems.

Die Nukleinsäuresequenzabschnitte A, B und C können verschiedenartiger Natur sein. Sie können z.B. ganz oder teilweise aus DNA, PNA (peptide nucleic acid) oder RNA bestehen. Dabei wird das Substrat in der Haarnadelschleife entsprechend der Spezifität der zu testenden Enzymaktivität ausgestaltet. Die Sequenzabschnitte können (semi)synthetischer oder rekombinanter Natur sein. Zur Herstellung rekombinanter Abschnitte vgl. allgemein Sambrook et al., "Molecular Cloning, A Laboratory Handbook", CSH Press, Cod Spring Harbor 1989. Im Einzelfall können die Abschnitte auch natürlichen Ursprungs sein. Die Abschnitte können, je nach Ausgestaltung des Substrats, einzeln hergestellt und dann verknüpft werden oder in einem Stück hergestellt werden.

In einer zusätzlich bevorzugten Ausführungsform des erfindungsgemäßen Verfahrens sind die Nukleinsäuresequenzabschnitte A B und C RNA oder DNA.

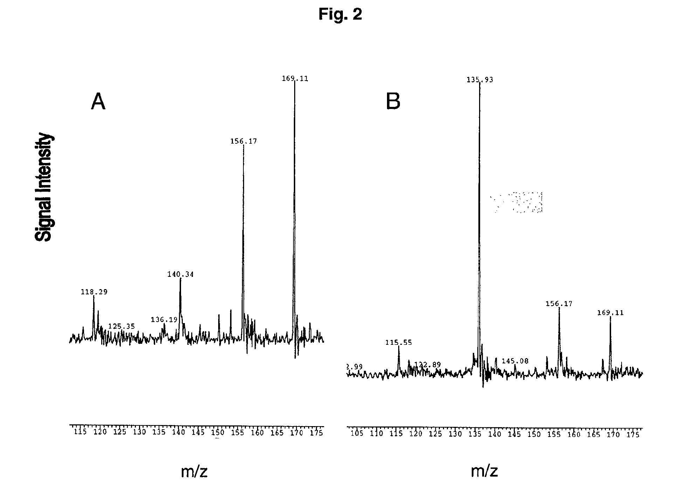

In einer anderen bevorzugten Ausführungsform des erfindungsgemäßen Verfahrens sind die Nukleinsäuresequenzabschnitte A B und C Hybride aus RNA und DNA.
Sofern die zu testende enzymatische Aktivität RNA spezifisch spaltet, wird bei der Konstruktion der Substrate (Fig.1) in einer bevorzugten Ausgestaltung der Erfindung für den Loop-Bereich RNA als Baustein beibehalten und lediglich der Stammbereich verändert. Um ein gegen Außeneinflüsse (Temperatur, Pufferzusammensetzung und pH-Wert) indifferentes Substrat zu erhalten, wird der Stammbereich als selbstkomplementäre DNA-Sequenz konzipiert (Beispiel 1). Dadurch ist es erstmals gelungen, RNase stabile Substrate als Hybride aufgebaut aus DNA- und RNA-Bausteinen darzustellen, welche über Monate stabil gelagert werden können und damit eine Voraussetzung für den Einsatz in der Wirkstoffanalytik erfüllen.
Die Basenabfolge der synthetischen Substrate wird entsprechend der erwarteten enzymatischen Aktivität konzipiert, z.B. wurde sie aus den von verschiedenen Autoren ermittelten Ricin A Erkennungsmotiven (Endo & Tsurugi, 1988; Orita et al., 1996; Chen et al., 1998) abgeleitet. Einige dieser synthetischen Oligonukleotide waren als Substrate für rRNA-N-Glycosidasen einsetzbar, was direkt durch den Einsatz von MALDI-MS Analytik gezeigt werden konnte (z.B. Fig. 2)

In einer weiteren bevorzugten Ausführungsform des erfindungsgemäßen Verfahrens ist die Aktivität, die zu einer sequenzspezifischen Depurinierung oder Depyrimidinierung einer Nukleinsäure ohne Strangbruch führt, eine N-Glycosidaseaktivität.
Bei der Reaktion von RNA mit N-Gycosidasen wird Adenin als Abspaltungsprodukt freigesetzt (Endo & Tsurugi, 1988). Für den Nachweis von Adenin stehen mehrere Möglichkeiten zur Verfügung.
Das abgespaltene Adenin (Mᵣ=135.93) wurde direkt über MALDI-TOF (MALDI-MS) nachgewiesen (Fig. 2 A: ohne Enzym; B: mit Enzym). Dieses Verfahren erlaubt zwar keine genaue Quantifizierung, kann jedoch als Beleg angeführt werden, daß neben dem im Loop befindlichen Adenin keine weiteren Abspaltungsprodukte freigesetzt wurden und damit davon ausgegangen werden kann, daß es sich hier um eine sehr spezifische Reaktion handelt. Zusätzlich wurden die Reaktionen in einem Polyacrylamid Gel unter denaturierenden Bedingungen (8 M Harnstoff) analysiert und densitometrisch ausgewertet (Daten nicht gezeigt). Die Substrate R28S sowie Y25S zeigten bei diesen Untersuchungen die stärksten Adenin-Abspaltungsbanden bei pH 7.2. Auch mit den Fluorophoren Fluorescein und Dabcyl markierte Substrate wurden erkannt und umgesetzt.

Die beschriebene Methode ist aufgrund der Sensitivität des Fluoreszenznachweises weiterhin geeignet, N-Glycosidase Aktivität in Wirkstoffen bis zu einer Konzentration von 1 bis 10 nM (bezogen auf rViscumin M: 57 kDa) nachzuweisen.

In einer besonders bevorzugten Ausführungsform des erfindungsgemäßen Verfahrens ist die N-Glycosidaseaktivität eine Typ-I oder Typ-II RIP Aktivität, AMP-Nucleosidase-, AMP-Desamidase-, Purin-Nucleosid-Phosphorylase- oder tRNA-Transglycosylase-Aktivität.

In einer bevorzugten Ausführungsform des erfindungsgemäßen Verfahrens ist die Probe eine biologische Probe.
Der Begriff "biologische Probe" bezeichnet im Sinne dieser Erfindung eine aus einer natürlichen Quelle stammende Probe. Hier sind u.a. zu nennen: Pflanzenbestandteile oder Pflanzenextrakte, Bodenproben, Pilz- und oder Bakterienlysate sowie tierische oder humane Proben.

In einer weiteren bevorzugten Ausführungsform des erfindungsgemäßen Verfahrens weisen die hybridisierenden Teile der Nukleinsäuresequenzabschnitte A und C eine Länge von 4-50 Nukleotiden auf.
Für die Analytik der Glycosidase-Aktivitäten kann die Tendenz eher zu kürzeren Nukleinsäuresequenzabschnitten führen. Test auf Endonukleaseaktivität muß jedoch mit längeren doppelsträngigen Nukleinsäuresequenzen durchgeführt werden, da die Restriktionsenzyme 5'-seitig einen Angriffspunkt zur Erkennung des Substrates benötigen. Dieses Erkennungsmotiv variiert in Abhängigkeit der gesuchten Aktivität (Enzym) und erklärt damit die Varianz in der Länge der hier angesprochenen Nukleinsäureabschnitte A und C (mindestens 10 Nukleotide). Die Erkennungssequenz (Palindrom) sollte dabei möglichst am "Fuß" des aus den Nukleinsäuresequenzabschnitten A und C bestehenden Stamms des Substrates liegen.

In einer bevorzugten Ausführungsform des erfindungsgemäßen Verfahrens weisen die hybridisierenden Teile der Nukleinsäuresequenzabschnitte A und C eine Länge von 5 bis 25 Nukleotiden und besonders bevorzugt eine Länge von 5-15 Nukleotiden auf.

In einer bevorzugten Ausführungsform des erfindungsgemäßen Verfahrens weisen die hybridisierenden Teile der Nukleinsäuresequenzabschnitte A und C palindromische Sequenzen auf, die als Erkennungssequenzen für Endonukleasen dienen.

In einer besonders bevorzugten Ausführungsform des erfindungsgemäßen Verfahrens weist die Nukleinsäuresequenzabschnitt B das Motif GAGA auf.
Diese Ausführungsform des erfindungsgemäßen Verfahrens ist besonders bevorzugt als RNA, da sie als Erkennungs- und Spaltungssequenz für verschiedene N-Glykosidasen wie Ricin oder Mistellektin dient.

In einer bevorzugten Ausführungsform des erfindungsgemäßen Verfahrens ist das Fluorophor-Quencher Paar 5-(2'-aminoehtyl)aminonaphtalin-1-Sulfonsäure (EDANS) 4-(4'-dimethylaminophenylazo) Benzoesäure (DABCYL), Fiuorescein/Rhodamin, Fluorescein/TAMRA, Fluorescein/Texas Red, Fluorescein/Rhodamin 6G, Fluorescein/Pyrenbutyrat, Fluorescein/Eosin, Fluorescein/Cy5, Fluorescein/Cy3, Anthranilamid/Nitrotyrosin, Fluorescein/DABCYL, Pyren/Pyren oder Coumarin/Ethidium.
Dabei zeichnet sich die Kombination Fluorescein/DABCYL als besonders bevorzugte Kombination aus, da bei niedrigem Hintergrundsignal ein optimaler Energietransfer gewährleistet ist.

In einer anderen bevorzugten Ausführungsform des erfindungsgemäßen Verfahrens sind Fluorophor und Quencher kovalent mit direkt miteinander hybridisierenden Nukleotiden verknüpft.

In einer zusätzlich bevorzugten Ausführungsform des erfindungsgemäßen Verfahrens ist das Fluorophor kovalent mit dem 5' Ende der einen DNA-Sequenz verbunden und der Quencher kovalent mit dem 3' Ende der anderen DNA-Sequenz verknüpft.

In einer zusätzlich bevorzugten Ausführungsform des erfindungsgemäßen Verfahrens ist das Agens ein Agens des Anilin-Typs.

In einer mehr bevorzugten Ausführungsform des erfindungsgemäßen Verfahrens ist das Agens des Anilin-Tpys ein wasserlösliches, am Aromaten elektrophil substituiertes Anilin.

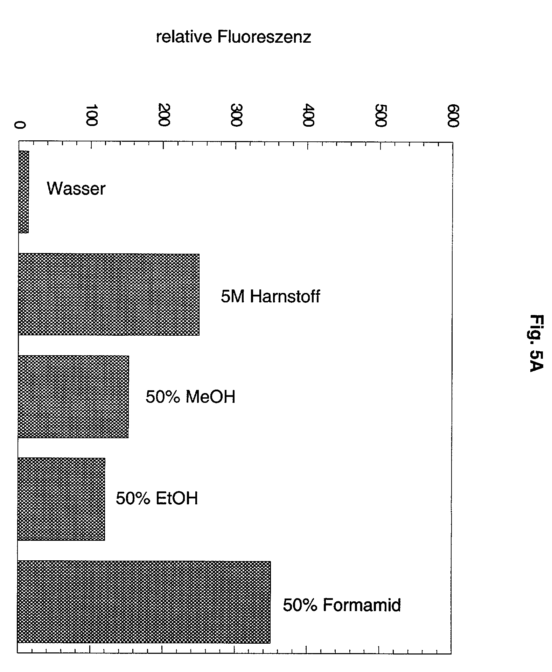

In einer ganz bevorzugten Ausführungsform des erfindungsgemäßen Verfahrens ist das wasserlösliche, am Aromaten elektrophil substituiertes Anilin 4-Aminohippursäure.
Die eigentliche Nachweisreaktion basiert wie bereits erwähnt auf der räumlichen Abkoppelung des Fluorophors vom Quencher. Sofern auf seuqenzspezifische Endonukleasen getestet wird, sind weitere chemische Manipulationen nicht erforderlich, wie oben erwähnt. Beim Nachweis enzymatischer Aktivitäten, die zu einer sequenzspezifischen Depurinierung oder Depyrimidierung führen, wird auf die chemisch induzierte Fragmentierung der markierten Substrate abgestellt. In der Literatur sind verschiedene Protokolle für die spezifische Spaltung deadenylierter RNA zu finden (Turchinsky et al., 1971; Peattie, 1979; Fernandez-Saiz et al. 1999; Carter et al., 1998). Allen Anleitungen ist die Verwendung von saurem Anilin gemeinsam (Beispiel 6). Die Übertragung dieser Protokolle auf das hier vorgestellte Detektionssystem ist zwar für den qualitativen Nachweis der Enzymaktivitäten möglich, war für quantitative Zwecke aber mit besonderen Schwierigkeiten verbunden. Zum einen konnte unter Verwendung der beschriebenen Protokolle keine reproduzierbare quantifizierbare Spaltungsreaktionen an den Substraten Y25S, E16S, OHS-1 und OHS-2 erhalten werden (Beispiel 6). Hinzu kam, daß sämtliche Fluoreszenzmessungen in Puffersystemen die Anilin enthielten, von starken Hintergrundsignalen begleitet waren, wodurch sich die gemessenen Signalzu Rausch Abstände (OHS-7, OHS-8 und OHS-9) deutlich verschlechterten. Dieses Problem lag zum Teil an dem erforderlichen pH-Wert. Unterhalb von pH 7 zeigt Fluorescein eine stetig mit dem pH-Wert abnehmende Fluoreszenz. Eine Umpufferung der in Anilin bei pH 4.0 durchgeführten Messungen auf pH-Werte oberhalb von 7 gelang nicht (Entmischung). Additive Lösungsmittelzusätze in den Puffer (Fig. 5A) unterdrückten zwar die Entmischung, erhöhten aber erneut die Hintergrundfluoreszenz der OHS. Dieser Einfluß von Additiven kann zum Teil auf ein Aufbrechen des Hairpin-Systems zurückgeführt werden (vgl. Fig 5A; 5M Harnstoff). Daher wurde nach einem Ersatz für Anilin als Spaltungsreagenz gesucht. In einem breit angelegten Screening nach einem das Anilin ersetzenden Spaltungsagenz wurden Polyamine (wie z.B. Spermin, Spermidin, Putrescin und Cadaverin); niedermolekulare Peptide; Interkalatoren (wie 9-Amino-Ellipticin, 3-Amino-Carbazol und 2,6-Diamino-Purin) und Enzyme wie die Exonuclease III untersucht. Zuletzt wurden auch noch Anilin-Analoga wie die 4-Aminohippursäure getestet.

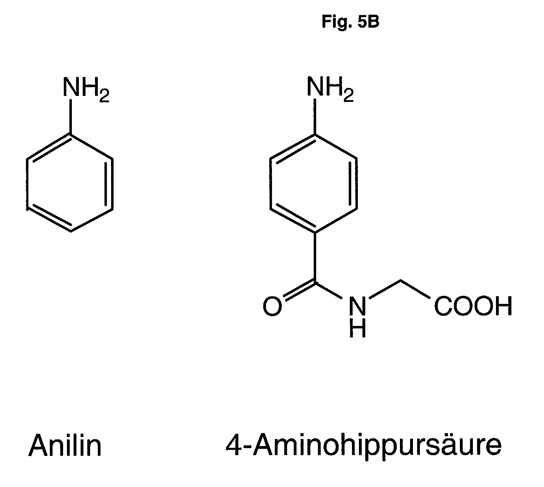

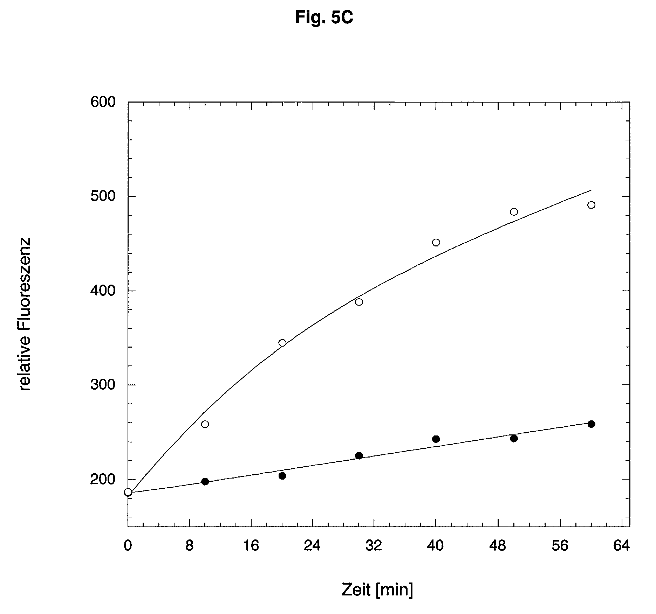

Bei dem breit angelegten Screening erwies sich überraschenderweise 4-Aminohippursäure (4-AHIP) als das am besten geeignete Spaltungsreagenz für OHS-Systeme (Fig. 5B). Mit 4-AHIP gelang die vollständig spezifische Fragmentierung aller hier eingesetzten Substrate. Als optimal erwies sich dabei eine Spaltungsdauer von 1 Stunde bei 65 °C (Fig. 5C). Im Gegensatz zu Anilin zeigt 4-AHIP keinerlei Löslichkeitsprobleme oberhalb von pH 4. Die gemessenen Hintergrundsignale blieben vor und nach Umpufferung stets niedrig. Mit 4-Aminohippursäure konnte damit ein ideales Spaltungsreagenz für die spezifische Fragmentierung deadenylierter Ribonukleinsäuren eingeführt werden (Fig. 5C offene Kreise). Eine zum Vergleich durchgeführte Substratspaltung mittels Anilin (gefüllte Kreise) zeigt einen deutlich flacheren Kurvenverlauf.

In einer bevorzugten Ausführungsform des erfindungsgemäßen Verfahrens erfolgen die Schritte (aa) und (ab) nacheinander.

Die Schritte (aa) und (ab) erfolgen in einer weiteren bevorzugten Ausführungsform des erfindungsgemäßen Verfahrens gleichzeitig.

In einer anderen bevorzugten Ausführungsform ist das erfindungsgemäßen Verfahrens ein Hochdurchsatzverfahren.
Um die Detektion und bevorzugt die Quantifizierung der Substratumsetzung auch unter Hoch-Durchsatz-Bedingungen (HTS) zu optimieren, werden beide freien Enden der chimären Oligonukleotidsequenz markiert. Als Marker dient am 5' Ende ein Flurophor (bevorzugt Fluorescein) und am 3' Ende ein Quencher (bevorzugt Dabcyl). Der Quencher wird dabei so ausgewählt, daß er die Anregungsenergie des Fluorophors größtenteils aufnimmt und nicht über eine Eigenfluoreszenz verfügt (FRET). Ein OHS wurde auch von Tyagi & Kramer 1996 als Beacon-System publiziert. In der vorliegenden Anmeldung wird jedoch im Gegensatz zu Tyagi & Kramer (1996) auf die Verwendung von Dansyl als Reportermolekül verzichtet, da diese Verbindung überraschenderweise eine starke, von der Polarität der Umgebung abhängige, Fluoreszenz aufweist. Anstelle des Dansyl wird in dem erfindungsgemäßen Verfahren als Reportermolekül bevorzugt das unter den spezifischen pH-Anforderungen stabilere Fluorescein eingesetzt.

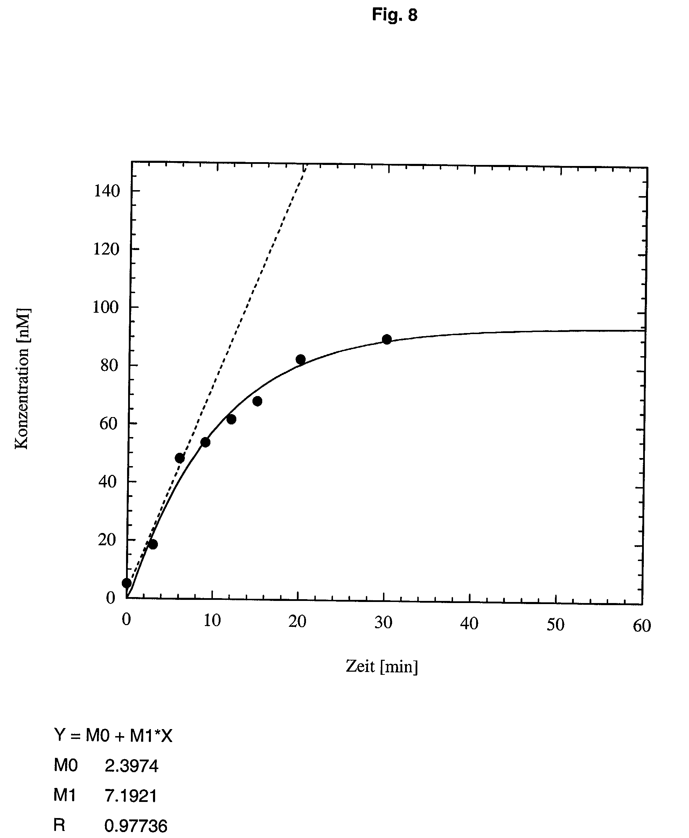

Ein weiterer wichtiger Aspekt für HTS ist, daß sich sämtliche Reaktionsschritte auch auf ein Mikrotiterplattenformat übertragen ließen (Beispiel 9). Hier wurde bei Einsatz von 10 nM rViscumin nach Quantifizierung für das Substrat OHS-8 eine Umsatzrate von 7.19 min⁻¹ ermittelt (Fig. 8). Diese Rate ist mit dem bei Einzelmessungen in der Küvette bestimmten Wert (10.05 min⁻¹) sehr gut vergleichbar (Fig. 7b). Die leichten Unterschiede in den beiden Zahlenwerten lassen sich auf Faktoren, die mit der Beschaffenheit der Plattenoberfläche zusammenhängen (z.B. Hydrophobizität oder ungenügendes Blockieren) zurückführen. Insgesamt erlaubt das entwickelte Testsystem einen schnellen Nachweis von z.B. N-Glycosidase Aktivität auch unter Hoch-Durchsatz-Bedingungen (HTS).

Weiterhin betrifft die vorliegende Erfindung die Verwendung eines Substrates wie oben beschrieben, zum Nachweis einer Substanz, welche die genannte Aktivität besitzt, in einer Probe.

Darüber hinaus betrifft die vorliegende Erfindung ein Kit enthaltend (a) ein Substrat, wie oben beschrieben; und (b) ein Agens, wie oben beschrieben.
Darüber hinaus kann der Kit entsprechend vorbereitete Reaktionsgefäße enthalten. Die einzelnen Reagenzien in den Reaktions- oder Aufbewahrungsgefäßen können ferner zusammen oder getrennt verpackt sein.

**Die Figuren zeigen:**
- **Fig. 1:**: Schematische Abbildung eines FRET-Substrat-Oligonukleotids für N-Glycosidasen
- **Fig. 2:**: MALDI-MS von umgesetztem Substrat. Das freigesetzte Adenin kann über den Massenpeak 135.93 (in Fig. 2 B) eindeutig nachgewiesen werden. In Fig. 2 A ist das Substrat ohne Zugabe des Enzyms inkubiert worden (Negativkontrolle)
- **Fig. 3:**: Emmissionsspektrum von gespaltenem Substrat OHS-8 zwischen 500 und 600 nm; Excitation bei 485 nm
- **Fig. 4:**: Eichreihe von chemisch quantitativ gespaltenem Substrat
- **Fig. 5A:**: Testung von Additiven bei der Spaltungsreaktion des depurinierten Substrates mittels Anilin
- **Fig. 5B:**: Strukturformeln der beiden Spaltungsreagenzien Anilin und 4-Aminohippursäure
- **Fig. 5C:**: Kinetik der Substratspaltung dargestellt mittels Fluoreszenzmessung
- **Fig. 6A:**: Schematische Darstellung der möglichen Versuchsaufbauten
- **Fig. 6B:**: Ermittlung der optimalen 4-Aminohippursäure Konzentration für die Substratspaltung
- **Fig. 6C:**: Ermittlung des geschwindigkeitsbestimmenden Schrittes der Substratumsetzung
- **Fig. 7A:**: Abhängigkeit des Substratumsatzes von der Enzymkonzentration
- **Fig. 7B:**: Auftragung des Substratumsatzes [nM/min] gegen die Enzymkonzentration [nM]
- **Fig. 8:**: Transfer des Tests in das MTP-Format

Der Offenbarungsgehalt der in dieser Spezifikation zitierten Dokumente wird hiermit per Referenz in die Spezifikation inkorporiert.

Die Beispiele erläutern die Erfindung.

### Beispiel 1: Aufbau der Oligonukleotide

Als Vorlage für die Substrate dienten unterschiedliche Erkennungsmotive für das Ricin A (Endo, 1988; Orita, 1996; Chen, 1998), die jedoch entscheidend verändert und optimiert wurden. Da es sich bei den in der Literatur beschriebenen Oligonukleotid-Sequenzen um reine Ribonukleinsäure-Moleküle handelt, wurde der Stammbereich bei den OHS als Desoxyribonukleinsäure entworfen, was hinsichtlich der Stabilität der Substrate in Lösung zu erheblichen Verbesserungen führte. Eine schematische Abbildung der hier entwickelten Substrate ist in Fig. 1 dargestellt. Bei der Konstruktion der Substrate für den Loop-Bereich wurden RNA-Nukleotide als Baustein beibehalten und lediglich der Stammbereich verändert. Um ein gegen Außeneinflüsse (Temperatur, Pufferzusammensetzung und pH-Wert) indifferentes Substrat zu erhalten, wurde der Stammbereich als selbstkomplementäre DNA-Sequenz konzipiert (Beispiel 1). Um eine anschließende Detektion zu ermöglichen, wurden beide Enden der chimären Oligonukleotidsequenz markiert. Als Marker diente am 5' Ende ein Flurophor (Fluorescein) und am 3' Ende ein Quencher (Dabcyl). Der Quencher wurde dabei so ausgewählt, daß er die Anregungsenergie des Fluorophors größtenteils aufnimmt und über keine Eigenfluoreszenz verfügt.

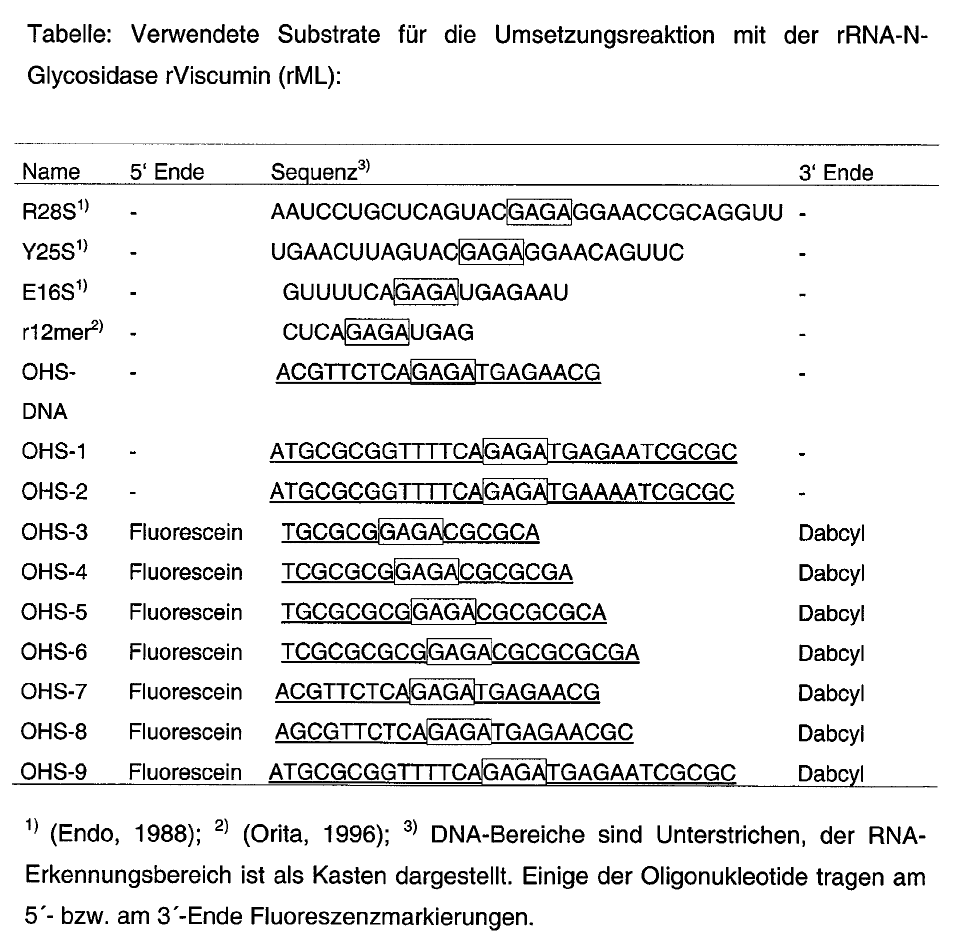

### Beispiel 2: Synthese und Aufreinigung der Oligonukleotide

Die Synthese sämtlicher Oligonukleotide erfolgte nach der Standard-Phosphoramidit Methode (Perseptive Biosystems). Alle verwendeten Monomere wurden in Konzentrationen von 0.1 M in trockenem Acetonitril (Wassergehalt unter 20 ppm) frisch gelöst. Zum Anbringen des 5' Fluoresceins (Perseptive Biosystems) wurden die Kupplungszeiten auf 5min verlängert. Nach der Synthese erfolgte die Entschützung der Basen und die Abspaltung vom Dabcyl-modifizierten Trägermaterial (Glen Research). Hierzu wurde der Träger in ein gasdichtes Schraubgefäß (Sarstaedt) überführt und 24 Stunden bei Raumtemperatur mit 400 µl konzentriertem Ammoniak (Merck) behandelt. Nach Filtration des Rohproduktes (Satorius) erfolgte die Aufreinigung der Oligonukleotide über RP-HPLC (20 Säulenvolumen, Material: Pharmacia 15 RPC, Puffer A: 10% Acetonitril/100 mM TEAA, Puffer B: 100% Acetonitril, Gradient: 0% A bis 70% B). Vor jeder präparativen Aufreinigung wurden analytische Vorläufe vorgenommen, um das genaue Elutionsverhalten zu bestimmen. Zur besseren Abtrennung wurde im Doppelwellenlängen Modus detektiert (455 nm und 495 nm). Die Hauptfraktionen wurden anschließend vereinigt und lyophilisiert. Die anschließende Entfernung der 2'TBDMS Schutzgruppe erfolgt mit 0.4 ml TBAF (Aldrich) über Nacht bei Raumtemperatur. Nach Abspaltung wurden die Oligonukleotide bei -80 °C mit 2 ml n-Butanol (Merck) und 20 µl 5 M Ammoniumacetat-Lösung (Fluka) 3 h gefällt, anschließend zentrifugiert (13.000 rpm) und getrocknet. Die fertigen Oligonukleotide wurden in 1ml sterilem Wasser aufgenommen und über die spezifische Absorption bei 260 nm quantifiziert. Die Lagerung erfolgte bei -20 °C.
Bei Oligonukleotiden, die keine Modifikation am 5' Ende tragen, wurde die letzte Trityl-Gruppe am 5' Ende der Sequenz nicht abgespalten. Die ebenfalls über RP-HPLC gereinigten Oligonukleotide (vgl. oben) wurden lyophilisiert und mit 0.3 ml 80% Essigsäure 45 min bei Raumtemperatur behandelt. Nach Abziehen der Essigsäure unter Vakuum wurde entsprechend dem oben angegebenen Protokoll fortgefahren.

### Beispiel 3: Substratumsetzung und Detektion des abgespaltenen Adenins

In verschiedenen Reaktionsansätzen (20 µl) wurde das Substrat R28S (0.5 µM) mit und ohne rViscumin (100 nM) in 1 x PBS (150 mM NaCI, 2.7 mM KCI, 6.7 mM Na₂HPO₄ und 1.9 mM KH₂PO₄ pH 7.2) 16 Stunden bei 37 °C inkubiert. Parallel dazu wurden Kontrollreaktionen ohne Substrat (mit Enzym) bzw. ohne Enzym (mit Substrat) durchgeführt. Für eine bessere Detektion im MALDI-TOF erfolgte eine Vorreinigung der Ansätze über RP-HPLC (Material: Pharmacia 15 RPC, Puffer A: 5% Acetonitril/100 mM TEAA, isokratisch). Die lyophilisierten Hauptfraktionen konnten direkt im Anschluß vermessen werden. In Fig. 2. ist erstmals das abgespaltene Adenin (Mᵣ=135.93) direkt über MALDI-TOF (MALDI-MS) nachgewiesen worden (Fig. 2 A: ohne Enzym; B: mit Enzym). Weitere unspezifische Abspaltungsprodukte konnten nicht identifiziert werden .

### Beispiel 4: Spaltungsdynamik der Substrate

Die Ermittlung der maximalen Fluoreszenzamplituden erfolgte nach chemischer Spaltung der Substrate mit NaOH. Hierzu wurde 100 µM des Oligonukleotids (OHS-8) in 0.5 N NaOH 45 min bei 90 °C inkubiert. Anschließend ließ man den Ansatz auf Raumtemperatur abkühlen und verdünnte mit dest. Wasser auf eine Endkonzentration von 1 µM. 10 µl dieses Spaltungsansatzes wurden mit 10 µl 1 M Tris-HCI versetzt, auf 100 µl mit dest. Wasser aufgefüllt und in einer Mikroküvette im Fluorimeter (Hitachi F-4500) vermessen. Das nicht gespaltene Oligonukleotid wurde entsprechend verdünnt und ebenfalls in 100 mM Tris-HCI pH 8.0 vermessen. Bei einer Anregungswellenlänge von 485 nm wurden Emissionsspektren im Bereich zwischen 500-600 nm aufgenommen (siehe Fig. 3). Das Differenzspektrum erbrachte bei allen verwendeten Substraten ein einheitliches Emissionsmaximum (ΔEₘₐₓ) um 523 nm. Die gefundenen Amplitudendifferenzen (F_{gespalten}-F_{ungespalten}) lagen je nach OHS zwischen 3 und 10 (523 nm).

### Beispiel 5: Korrelation der eingesetzten Substratmenge mit dem Fluoreszenzsignal

Die Quantifizierung der umgesetzten Substratmenge kann über die Messung einer zuvor vollständig gespaltenen Referenzprobe erfolgen. Zu diesem Zweck wurden bei allen verwendeten Substraten alkalische Spaltungen, entsprechend der Anleitung im Beispiel 4, durchgeführt. Steigende Mengen der so erhaltenen Stammlösung wurden mit 10x Enzympuffer (1.6 M KCI, 400 mM Citrat pH 3.0 und 8 mM MgCl₂) und dest. Wasser weiter verdünnt. 10 µl jeder Verdünnung werden 40 µl 100 mM 4-Aminohippursäure (Fig. 5B) pH 4.0 (Merck) zugesetzt und 1 h bei 65 °C inkubiert. Anschließend wurden weitere 50 µl 1 M Tris-HCI jedem Ansatz zugesetzt und nach kurzer Zentrifugation die Fluoreszenz (E_{X} = 485 nm und E_{M} = 523 nm) direkt in einer Mikroküvette ermittelt. Die erhaltenen Ergebnisse wurden anschließend gegen die Konzentration aufgetragen (Fig. 4). Die über Regression bestimmte Geradengleichung diente als Grundlage für die Umrechnung der in Umsetzungsreaktionen mit N-Glycosidasen ermittelten Fluoreszenzwerte in die erhaltenen Produktkonzentrationen.

### Beispiel 6: Spaltung der deadenylierten Produkte

In der Literatur sind verschiedene Protokolle für die spezifische Spaltung deadenylierter RNA zu finden. Allen Anleitungen ist die Verwendung von saurem Anilin (Endo, 1987) gemeinsam. Anilin (Sigma) wurde vor Gebrauch frisch unter Vakuum destilliert. 9.3 g Anilin (1.02 g/ml) wurden mit 70 ml dest. Wasser verdünnt und solange mit konzentrierter Essigsäure (Fluka) versetzt, bis sich beide Phasen miteinander vollständig vermischten. Anschließend wurde auf 100 ml mit dest. Wasser aufgefüllt und der pH-Wert auf 4.2 eingestellt.
Für die Spaltungsreaktion (50µl Ansatz) wurden die deadenylierten Oligonukleotide (0.5 µM) mit 2% LiClO₄ (Aldrich) in Aceton (Fluka) auf Eis gefällt (500 µl). Nach Zentrifugation (13000 rpm) und Vakuumtrocknung erfolgte die Spaltungsreaktion in 50 µl 1 M Anilinlösung (vgl. oben). Der Ansatz wurde hierzu 15 min bei 65 °C inkubiert und anschließend mit 500 µl kaltem Aceton (Fluka) gefällt. Nach erneuter Vakuumtrocknung wurden die Proben direkt auf ein Gel aufgetragen.
Oberhalb von pH 4.2 entmischten sich Anilin und Wasser. Damit auch bei höheren pH-Werten direkt im Spaltungsansatz (ohne Acetonfällung) gemessen werden konnte, wurden unterschiedliche Zusätze für die Umpufferung ausprobiert. Neben Methanol (50% Methanol, 1 M Tris-HCI pH 8.0) bzw. Ethanol (50% Ethanol, 1M Tris-HCI pH 8.0) diente auch Formamid (50% Formamid, 1 M Tris-HCI pH 8.0) als Additiv. 50 µl dieser Lösungen wurden direkt in den Spaltungsansatz (50 µl) zugegeben, gut vermischt und anschließend am Fluorimeter vermessen (Fig. 5A).

Als alternatives Spaltungsreagenz wurde 4-Aminohippursäure erfolgreich eingesetzt. 1.94 g 4-Aminohippursäure (Merck) wurden in 70 ml dest. Wasser aufgenommen und solange mit 4 N KOH (Fluka) versetzt, bis bei etwa pH 4.0 die 4-Aminohippursäure sich vollständig in Wasser aufgelöst hatte. Das Volumen wurde anschließend auf 100 ml mit dest. Wasser aufgefüllt und der pH-Wert erneut auf 4.0 nachjustiert. Zur Überprüfung der Fähigkeiten von 4-Aminohippursäure, die Oligonukleotide an der depurinierten Stelle zu spalten, diente ein über Nacht inkubierter Spaltungsansatz (40 mM Citratpuffer pH 3.0, 160 mM KCI, 0.8 mM MgCl₂, 0.5 µM Substrat-OHS sowie 100 nM Mistellektin). Je 10 µl dieses Ansatzes wurden mit 40 µl 100 mM 4-Aminohippursäure (vgl. oben) versetzt und unterschiedlich lange (0 min - 60 min) bei 37 °C und 65 °C inkubiert. Nach Spaltung durch 4-Aminohippursäure wurde jeder Ansatz mit 50 µl 100 mM Citratpuffer (pH 4.0) versetzt und am Fluorimeter direkt vermessen (E_{X} = 485 nm bzw. E_{M} = 523 nm)( Siehe Fig. 5C).

### Beispiel 7: Etablierung eines Meßverfahrens für den Nachweis der N-Glycosidase Aktivität

Die markierten Substrate ließen sich ganz unterschiedlich in einem Assay einsetzen. Zum einen konnte die Fragmentierungsreaktion direkt in Gegenwart der N-Glycosidase durchgeführt werden. Der Fluoreszenzanstieg ließ sich hierbei direkt verfolgen (*online*). Dem gegenüber stand der Einsatz von 4-Aminohippursäure nach enzymatischer Umsetzung (*postenzymatisch*). Wichtig für den simultanen Einsatz von N-Glycosidase und Spaltungsreagenz war zunächst der Einfluß unterschiedlicher 4-Aminohippursäure-Konzentrationen (5 mM, 12.5 mM, 25 mM und 50 mM) auf die *online* Spaltungskinetik (10 nM rViscumin) des Substrats OHS-7 (0.2 µM) bei pH 3.0 (40 mM Citrat pH 3.0, 160 mM KCI und 0.8 mM MgCl₂. Wie gezeigt werden konnte, erreichte die beobachtete Umsetzungsreaktion ein Maximum in Gegenwart von 12.5 mM 4-Aminohippursäure. Wichtig bei der Verwendung zur *online* Detektion ist ferner der geschwindigkeitslimitierende Schritt innerhalb der Gesamtreaktion. Dieser wurde in zwei getrennten Ansätzen näher untersucht. Im ersten Ansatz (40 mM Citrat pH 3.0, 160 mM KCI und 0.8 mM MgCl₂) wurde die Fragmentierungs- bzw. Deadenylierungsgeschwindigkeit des Substrats OHS-7 (0.2 µM) in Gegenwart von 10 nM Mistellektin und 12.5 mM 4-Aminohippursäure gleichzeitig gemessen. In einem zweiten Ansatz wurde das über Nacht mit Mistellektin (10 nM) deadenylierte Substrat OHS-7 (0.2 µM) direkt mit 12.5 mM 4-Aminohippursäure umgesetzt (40 mM Citrat pH 3.0, 160 mM KCI und 0.8 mM MgCl₂). Die vergleichenden Messungen belegten, daß in der *online* Detektion die Fragmentierungsreaktion geschwindigkeitslimitierend für die Gesamtreaktion ist. Für kinetische Untersuchungen eignet sich daher die *online* Detektion nicht.

### Beispiel 8: Kinetische Messungen

Für kinetische Messungen wurde das Substrat OHS-8 verwendet. In einem Eppendorf-Gefäß wurden bis auf das rViscumin sämtliche Komponenten (90 µl: 40 mM Citrat pH 3.0, 160 mM KCI, 0.8 mM MgCl₂ und 333 nM OHS-8) auf Eis pipettiert. Anschließend wurde 10 µl rViscumin (40 mM Citrat pH 3.0, 160 mM KCI, 0.8 mM MgCl₂ und 50, 100 sowie 200 nM) zugesetzt und bei 37°C inkubiert. Nach unterschiedlichen Zeiten (0 min-14 min) wurden jeweils 10 µl Probe entnommen und unmittelbar in 40 µl eiskaltes 100 mM 4-Aminohippursäure überführt. Die Spaltungsansätze wurden anschließend 1 h bei 65 °C inkubiert. Nach Abkühlen auf Raumtemperatur wurden alle Ansätze mit 50 µl 1 M Tris-HCI pH 8.0 versetzt und am Fluorimeter vermessen (E_{X} = 485 nm bzw. E_{M} = 523 nm).

### Beispiel 9: Belegung der HTS-Tauglichkeit des Verfahrens

Zum Beleg der Hoch-Durchsatz-Tauglichkeit des Meßverfahrens wurden die Versuche auch auf Mikrotiterplatten-Format überführt. Alle Messungen ließen sich auch in schwarzen 96'er Mikrotiterplatten (Corning) durchführen. Vor der eigentlichen Reaktion wurde die hydrophobe Oberfläche der Platten über Nacht mit 200 µl SuperBlock behandelt (Pierce) und zweimal mit 200 µl dest. Wasser ausgewaschen. Die entsprechenden Kavitäten wurden mit 40 µl 100 mM 4-Aminohippursäure gefüllt und auf Eis gestellt.
Die enzymatische Umsetzung erfolgte außerhalb der Platten. In einem Eppendorf-Gefäß wurden bis auf das rViscumin sämtliche Komponenten (90µl: 40mM Citrat pH 3.0, 160 mM KCI, 0.8 mM MgCl₂ und 333 nM OHS-8) auf Eis pipettiert. Anschließend wurde 10 µl Mistellektin (40 mM Citrat pH 3.0, 160 mM KCI, 0.8 mM MgCl₂ und 100 nM) zugesetzt und bei 37 °C inkubiert. Zur kinetischen Betrachtung der Reaktion wurden zu variierenden Zeiten jeweils 10 µl Probe entnommen und unmittelbar in die Mikrotiter-Kavitäten überführt. Die gesamten Platten werden dicht verschlossen (Plate Sealer von Corning) und 1 h bei 65 °C inkubiert. Nach Abkühlen auf Raumtemperatur wurde in alle Kavitäten 50 µl 1 M Tris-HCI pH 8.0 pipettiert. Die Platten konnten direkt im Anschluß am Fluoreszenzreader (FLUOstar BMG LabTechnologies) vermessen werden (E_{X} = 485 nm; E_{M} = 520 nm).

### Literatur:

Barbieri, L., Bolognesi, A., Cenini, P., Falasca, A. I., Minghetti, A., Garofano, L., Guicciardi, A., Lappi, D., Miller, S.P. und Stirpe, F. (1989) *Biochem. J*. **257,** 801-807
Barbieri, L., Battelli, M.G., und Stirpe, F. (1993) *Biochim*. *Biophys*. *Acta* **1154,** 237-282
Barbieri, L., Valbonesi, P., Gorini, P., Pession, A. und Stirpe, F. (1996) *Biochem*. *J.* **319,** 507-513
Barbieri, L., Valbonesi, P., Bonora, E., Gorini, P., Bolognesi, A. und Stirpe, F. (1997) *Nucl*. *Acids Res.* **25,** 518-522
Beuth, J., Ko, H.L., Gabius, H.-J., Burrichter, H., Oette, K., and Pulverer, G. (1992): *Clin*. *Investig*. **70,** 658-661
Brigotti, M., Barbieri, L., Valbonesi, P., Sirpe, F., Montanaro, L. und Sperti, S. (1998) *Nucl*. *Acids Res.* **26,** 4306-4307
Carter, P.J., Breiner, K.M. Thorp, H.H. (1998) *Biochemistry* **37,** 13736-13743
Chen, X. Y., Link, T.M. und Schramm V.L. (1998) *Biochemistry* **37,** 11605-11613
Endo, Y., Glück, A. und Wool, I.G. (1991) *J. Mol*. *Biol.* **221,** 193-207
Endo Y., und Tsurugi, K. (1987a) *J*. *Biol*. *Chem.* **262,** 5908-5912
Endo, Y., und Tsurugi, K. (1987b) *J*. *Biol*. *Chem.* **262**, 8128-8130
Endo, Y., und Tsurugi, K. (1988) *J*. *Biol*. *Chem.* **263**, 8735-8739
Fernandez-Saiz, M., Henderson, P.T., Wilson, W.D., and Schuster, G.B. (1999): *Photochem*. *Photobiol*. **70,** 847-852
Franz, H., Kindt, A., Ziska, P. Bielka, H., Benndorf, R. und Venker, L. (1982) *Acta Biol*. *Med*. *Ger*. **41,** K9-K16
Gasperi-Campani, A., Barbieri, L. Morelli, P. und Stirpe, F., (1980) *Biochem*. *J*. **186,** 439-441
Ghosh S.S., Eis, P.S., Blumeyer, K., Fearon, K, and Millar, D.P. (1994) Nucl. Acids Res. 22, 3155-3159
Goupaul, D.N., Meyer, S.L., Degano M., Sacchettini J.C., Schramm, V.L. *Biochemistry* **35,** 5963-5970
Glück, A., Endo, Y. und Wool, I.G. (1992) *J*. *Mol*. *Biol.* **226,** 411-424 Hajto, T., Hostanska, K., and Gabius, H.-J. (1989): *Cancer Res.* **49**, 4803-4808
Hershfield, M.S. uns Mitchell, B.S. (1995) In: The metabolic basis of inherited Disease, edn 7, Edts: Scriber, C.R. et al.; pp 1725-1768
Huang, Q., Liu, S., Tang, Y., Jin S., Wang, Y (1995) *Biochem*. *J.* **309,** 285-298
Kline, P.C. und Schramm, V.L. (1993) *Biochemistry* **32,** 13212-13219
Krawetz, J.E. und Boston, R.S. (2000) *Eur*. *J. Biochem.* **267,** 1966-1974
Langer, M., Rothe, M, Eck J., Möckel, B., und Zinke, H. (1996) *Anal. Biochem.* **243,** 150-153
Link, T., Chen, X.Y., Niu, L.H. und Schramm, V.L. (1996) *Toxicon,* **34,** 1317-1324
Mentch, F., Parkin, D.W., Schramm, V.L. (1987) *Biochemistry* **26,** 921-930
Meyer, S.L., Kvalnes-Krick, K.L., Schramm V.L. (1989) *Biochemistry* **28,** 8734-8743
Moazed, D., Stern, S. und Noller, H.F. (1986) J. *Mol*. *Biol.* **187,** 399-416
Möckel, B., Schwarz, T., Zinke, H., Eck, J., Langer, M., and Lentzen, H.: (1997) *Drug Res.* **47,** 1145-1151
Monzingo, A.F., und Robertus, J.D. (1992) *J. Mol*. *Biol*. **227,** 1136-1145
Olson, M. (1997) *Proteins: Structure*, *Function*, *and Genetics* **27**, 80-95
Orita M., Nishikawa F., Khono T., Senda T., Mitsui Y., Endo Y., Taira K. and Nishikawa S. (1996) *Nucl*. *Acids Res.* **24,** 611-618
Peattie, D.A. (1979): *Proc*. *Natl*. *Acad*. *Sci*. *USA* **76,** 1760-1764
Pelham, H. R. B. und Jackson, R. J. (1976) *Eur*. *J. Biochem.* **67,** 247-256
Romier, C., Reuter, K., Such, D., Ricne, R. (1996) *EMBO J*. **15,** 2850-2857
Sambrook et al. (1989), "Molecular Cloning, A Laboratory Manual", CSH Press, Cold Spring Harbor
Skinner, L. M. und Jackson, M. P. (1997) J. *Bacteriol.* **179,** 1368-1374
Turchinsky, M.F., Guskova, L.I., Hazai, I., Budowsky, E.I, Kochetkov, N.K. (1971) *Biochim*. *Biophys*. *Acta* **254,** 366-379
Tyagi, S. (1996) *Nature Biotechnology,* **14,** 303-308
Zamboni, M., Brigotti, M., Rambelli, F., Montanaro, L. und Sperti, S. (1989) *Biochem*. *J*. **259,** 639-643

## Patentansprüche

1. Verfahren zum Nachweis einer Substanz mit einer enzymatischen Aktivität, die zu einer sequenzspezifischen Depurinierung oder Depyrimidinierung einer Nukleinsäure ohne Strangabbruch führt, oder mit einer sequenzspezifischen Endonucleaseaktivität, in einer Probe, wobei
(a) die Probe
(aa) mit einem Substrat in Kontakt gebracht wird, das
(i) drei Nukleinsäuresequenzabschnitte (A, B und C) aufweist, wobei der Nukleinsäuresequenzabschnitt B zwischen den Abschnitten A und C liegt, die Nukleinsäuresequenzabschnitte A und C Sequenzen aufweisen, die komplementär zueinander sind und somit unter physiologischen Bedingungen miteinander hybridisieren und dadurch das Substrat eine Haarnadelstruktur ausbildet und wobei der Nukleinsäuresequenzabschnitt B oder die Nukleinsäureabschnitte A und C nach Hybridisierung ein Erkennungsmotif für die Aktivität enthalten; und
(ii) (ii) ein Fluorophor-Quencher-Paar aufweist, wobei das Fluorophor kovalent mit Sequenzabschnitt A oder C verbunden ist und der Quencher kovalent mit dem anderen Sequenzabschnitt (C oder A) verknüpft ist und wobei Fluorophor und Quencher sich bei der Hybridisierung der DNA-Sequenzen in räumlicher Nähe zueinander befinden, so daß die vom Fluorophor emittierte Lichtenergie vom Quencher gequencht wird; und
(ab) sofern die Substanz eine Substanz mit einer enzymatischen Aktivität ist, die zu einer sequenzspezifischen Depurinierung oder Depyrimidinierung einer Nukleinsäure ohne Strangbruch führt, mit einem Agens in Kontakt gebracht wird, das das Substrat spezifisch an einer Nukleotidposition im Bereich des Nukleinsäuresequenzabschnitts B spaltet, die durch die Substanz depuriniert oder depyrimidiniert wird; und
(b) anhand der Emission der Fluorophor-spezifischen Lichtenergie ermittelt wird, ob eine Substanz mit der genannten Aktivität in der Probe vorhanden ist.

2. Verfahren nach Anspruch 1, wobei der Nachweis ein qualitativer Nachweis ist.

3. Verfahren nach Anspruch 1, wobei der Nachweis ein quantitativer Nachweis ist.

4. Verfahren nach einem der Ansprüche 1 bis 3, wobei die Nukleinsäuresequenzabschnitte A B und C RNA oder DNA sind.

5. Verfahren nach einem der Ansprüche 1 bis 3, wobei die Nukleinsäuresequenzabschnitte A B und C Hybride aus RNA und DNA sind.

6. Verfahren nach einem der Ansprüche 1 bis 5, wobei die Aktivität, die zu einer sequenzspezifischen Depurinierung oder Depyrimidinierung einer Nukleinsäure ohne Strangbruch führt, eine N-Glycosidaseaktivität ist.

7. Verfahren nach Anspruch 6, wobei die N-Glycosidaseaktivität eine Typ-I oder Typ-II RIP Aktivität, AMP-Nucleosidase-, AMP-Desamidase-, Purin-Nucleosid-Phosphorylase- oder tRNA-Transglycosylase-Aktivität ist.

8. Verfahren nach einem der Ansprüche 1 bis 7, wobei die Probe eine biologische Probe ist.

9. Verfahren nach einem der Ansprüche 1 bis 8, wobei die hybridisierenden Teile der Nukleinsäuresequenzabschnitte A und C eine Länge von 4-50 Nukleotiden aufweisen.

10. Verfahren nach Anspruch 9, wobei die hybridisierenden Teile der Nukleinsäuresequenzabschnitte A und C eine Länge von 5 bis 25 Nukleotiden aufweisen.

11. Verfahren nach einem der Ansprüche 1 bis 10, wobei die hybridisierenden Teile der Nukleinsäuresequenzabschnitte A und C palindromische Sequenzen aufweisen.

12. Verfahren nach einem der Ansprüche 4 bis 11, wobei die Nukleinsäuresequenzabschnitt B das Motif GAGA aufweist.

13. Verfahren nach einem der Ansprüche 1 bis 12, wobei das Fluorophor-Quencher Paar z.B.: 5-(2'-aminoehtyl)aminonaphtalin-1-Sulfonsäure (EDANS), 4-(4'-dimethylaminophenylazo) Benzoesäure (DABCYL), Fluorescein/Rhodamin, Fluorescein/TAMRA, Fluorescein/Texas Red, Fluorescein/Rhodamin 6G, Fiuorescein/Pyrenbutyrat, Fluorescein/Eosin, Fluorescein/Cy5, Fluorescein/Cy3, Anthranilamid/Nitrotyrosin, Fluorescein/DABCYL, Pyren/Pyren oder Coumarin/Ethidium ist.

14. Verfahren nach einem der Ansprüche 1 bis 13, wobei Fluorophor und Quencher kovalent mit direkt miteinander hybridisierenden Nukleotiden verknüpft sind.

15. Verfahren nach einem der Ansprüche 1 bis 22, wobei das Fluorophor kovalent mit dem 5' Ende der einen DNA-Sequenz verbunden ist und der Quencher kovalent mit dem 3' Ende der anderen DNA-Sequenz verknüpft ist.

16. Verfahren nach einem der Ansprüche 1 bis 15, wobei das Agens ein Agens des Anilin-Typs ist.

17. Verfahren nach Anspruch 16, wobei das Agens des Anilin-Tpys ein wasserlösliches, am Aromaten elektrophil substituiertes Anilin ist.

18. Verfahren nach Anspruch 17, wobei das wasserlösliche, am Aromaten elektrophil substituiertes Anilin 4-Aminohippursäure ist.

19. Verfahren nach einem der Ansprüche 1 bis 18, wobei die Schritte (aa) und (ab) nacheinander erfolgen.

20. Verfahren nach einem der Ansprüche 1 bis 18, wobei die Schritte (aa) und (ab) gleichzeitig erfolgen.

21. Verfahren nach einem der Ansprüche 1 bis 20, das ein Hochdurchsatzverfahren ist.

22. Verwendung eines Substrates, wie in einem der Ansprüche 1 bis 21 dargestellt, zum Nachweis einer Substanz, welche die genannte Aktivität besitzt, in einer Probe.

23. Kit enthaltend
(a) ein Substrat, wie in einem der Ansprüche 1 bis 21 dargestellt; und
(b) ein Agens, wie in einem der Ansprüche 1 bis 21 dargestellt.
